# EUROPÄISCHE PATENTSCHRIFT

(11) **EP 2 364 643 B1**
(45) Veröffentlichungstag und Bekanntmachung des Hinweises auf die Patenterteilung: **22.07.2015**
(21) Anmeldenummer: 11155180.0
(22) Anmeldetag: 21.02.2011
(51) Int. Cl.: A61B 5/053, A61B 5/0295, A61B 5/029

(54) **Elektromedizinisches Implantat und Überwachungssystem mit dem elektromedizinischen Implantat**
Electromedical implant and monitoring system comprising the electromedical implant
Implant électromédical et système de surveillance doté de cet implant électromédical

(30) Priorität: 09.03.2010 US 311789 P
(43) Veröffentlichungstag der Anmeldung: 14.09.2011
(73) Patentinhaber: BIOTRONIK SE & Co. KG, 12359 Berlin (DE)
(72) Erfinder: Czygan, Gerald, 91054, Buckenhof (DE); Lippert, Michael, 91522, Ansbach (DE); Skerl, Olaf, 18209, Bad Doberan (DE)
(74) Vertreter: Lindner-Vogt, Karin L.

(56) Entgegenhaltungen:
- EP-A1- 2 149 336
- EP-A2- 1 665 983
- DE-A1- 10 061 189
- DE-A1-102005 042 041
- US-A1- 2008 183 083
- US-A1- 2009 062 667

## Beschreibung

Die Erfindung betrifft ein elektromedizinisches Implantat zum Überwachen eines kardialen Blutstroms und eines epithorakalen, peripheren Blutstroms eines Lebewesens. Weiter betrifft die Erfindung ein Überwachungssystem mit dem elektromedizinischen Implantat.

Ein elektromedizinisches Implantat der eingangs genannten Art kann ein reines Monitoringimplantat oder ein Monitoring- und Therapieimplantat sein. Als so genanntes Monitoringimplantat - im Unterschied zu Therapieimplantaten, wie implantierbare Herzstimulatoren oder dergleichen - hat ein elektromedizinisches Implantat zum Überwachen von Blutströmen eines Lebewesens (Patienten) keine Therapieoptionen. Ein Monitoringimplantat kann auch für Patienten ohne Therapieindikation sinnvoll und wichtig sein, um Blutströme des Patienten zu überwachen. So kann sich beispielsweise erst aus einer längerfristigen Überwachung des Patienten mit dem Monitoringimplantat eine Indikation zur Elektrotherapie ergeben bzw. eine Art der Elektrotherapie im Vorfeld derselben genauer spezifizieren lassen. Dabei hat sich insbesondere die Überwachung eines kardialen Blutstroms und eines epithorakalen, peripheren Blutstroms eines Lebewesens als zweckmäßig erwiesen, um eine sogenannte Pulswellenlaufzeit zu bestimmen. Unter einer Pulswellenlaufzeit ist die Zeitdifferenz zwischen einem Zeitpunkt eines Blutauswurfs aus dem Herzen eines Patienten bis zum Zeitpunkt eines Blutpulses im thorakalen Gewebe zu verstehen, wobei der Blutpuls im thorakalen Gewebe zeitlich verzögert erfolgt und durch den Blutauswurf verursacht ist.

Zur Bestimmung der Pulswellenlaufzeit sind unterschiedliche Ansätze aus dem Stand der Technik bekannt. So ist aus US 7,029,447 ein nicht-invasives System zur Messung des Blutdrucks eines Patienten bekannt, wobei mit einem körperexternen Messgerät die Pulswellenlaufzeit aus der Differenz der Zeitpunkte der mechanischen Herzkontraktion und der Ankunft der Pulswelle in der Peripherie bestimmt wird. Der Zeitpunkt der Herzkontraktion wird aus der extern gemessenen Thoraximpedanz und dem QRS-Komplex eines Oberflächen-EKGs bestimmt. Die Ankunft der Pulswelle in einem peripheren Organ, nämlich dem Unterarm, wird mittels der optischen Plethysmografie ermittelt.

US 6,748,262 und US 5,743,856 beschreiben weitere nicht-invasive Verfahren zur Bestimmung der Pulswellenlaufzeit anhand eines EKG-Signals und eines thorakalen Impedanzsignals.

Solche nicht-invasiven Systeme erweisen sich als zwar sinnvoll zur Realisierung eines Monitoringimplantats, jedoch sind diese nachteilig und gegebenenfalls umständlich in der Handhabung beim Überwachen eines kardialen Blutstroms und eines epithorakalen peripheren Blutstroms zur Bestimmung der Pulswellenlaufzeit über einen längeren Zeitraum.

Andererseits sind kardial-invasive Systeme keine Option für Patienten ohne Therapieindikation. Die Implantation von Sonden im Herz, beispielsweise zur Bestimmung eines EKG-Signals mit einem Herzstimulator, ist mit Risiken für den Patienten behaftet und aufwändig. Insofern ist eine an sich bekannte kardial-invasive Thoraximpedanzmessung mittels im Herzen implantierbarer Sonden eines Herzstimulators für ein Impedanzkardiogramm (ICG) oder ein Elektrokardiogramm (ECG) nur für Patienten mit einer Herzinsuffizienz angemessen, jedoch keine Option für Patienten ohne Indikation zur Elektrotherapie. Gleichwohl bildet diese Gruppe von Patienten einen signifikanten Anteil von Patienten mit Herzinsuffizienz.

Wünschenswert ist ein elektromedizinisches Implantat zum Überwachen eines kardialen Blutstroms und eines epithorakalen peripheren Blutstroms eines Lebewesens (Patienten), mit dem eine Pulswellenlaufzeit auch langfristig überwacht werden kann, ohne dass eine kardiale Invasivität geboten ist.

Ein dazu eingangs genanntes elektromedizinisches Implantat ist beispielsweise aus US 2009/0062667 A1 bekannt, bei welchem letztlich zur Überwachung eines arteriellen Blutdrucks eine Pulswellenlaufzeit aus Pulsspitzenankunftszeiten zweier Signale bestimmt wird. Mittels subkutan implantierten Elektroden wird ein erstes Signal in Form eines Elektrokardiogramms ermittelt, welches indikativ für eine elektrische Aktivität eines Patientenherzens, nämlich eines Blutauswurfs aus dem Herzen ist. Zusätzlich wird ein pektoral implantierter Sensor in Form eines Photoplethysmografiesensors genutzt, um ein entsprechendes Signal zu ermitteln, welches indikativ für eine mechanische Aktivität eines Patientenherzens, nämlich die pektorale Ankunft eines Blutpulses, ist. Dieses Konzept kann als ein implantierbarer Monitor ohne Elektrotherapievorrichtung realisiert werden. Die Signale eines Elektrokardiogramms der subkutanen Elektroden und eines pektoralen Photoplethysmografiesensors werden im Rahmen einer Auswerteeinheit genutzt, um einen arteriellen Blutdruck zu bestimmen.

Während sich die Messung einer elektrischen Aktivität und einer mechanischen Aktivität eines Patientenherzens als grundsätzlich sinnvoll zur Bestimmung einer Pulswellenlaufzeit in einem Monitoringimplantat erweist, so ist dieser Ansatz dennoch verbesserungswürdig.

Der Erfindung liegt die Aufgabe zugrunde, ein elektromedizinisches Implantat, insbesondere ein reines Monitoringimplantat, zum Überwachen eines kardialen Blutstroms und eines epithorakalen peripheren Blutstroms eines Lebewesens anzugeben, mit dem auf vergleichsweise einfache und dennoch verlässliche Weise jedenfalls ein dem epithorakalen peripheren Blutstrom zugeordnetes Messsignal ermittelt werden kann. Aufgabe der Erfindung ist es auch, ein Überwachungssystem unter Nutzung des elektromedizinischen Implantats anzugeben, mit dem wenigstens die Pulswellenlaufzeit, insbesondere aus der Pulswellenlaufzeit ableitbare weitere Patientenwerte, dauerhaft bzw. über einen längeren Zeitraum überwachbar und abrufbar sind.

Betreffend das elektromedizinische Implantat wird die Aufgabe durch die Erfindung mittels eines elektromedizinischen Implantats der eingangs genannten Art gelöst, das erfindungsgemäß aufweist:
- eine Detektoranordnung (10, 10', 10") zum Erfassen eines dem kardialen Blutstrom (B1) zugeordneten ersten Messsignals (S1) und eines dem epithorakalen, peripheren Blutstrom (B2) zugeordneten zweiten Messsignals (S2);
- eine an der Detektoranordnung (10, 10`, 10") angeschlossene Überwachungsanordnung (20), die
   eingerichtet ist, einen ersten Parameter (P1) aus dem ersten Messsignal (S1) zu erzeugen, der indikativ für einen Zeitpunkt (t_{sys}) eines Blutauswurfs aus dem Herzen ist, und eingerichtet ist, einen zweiten Parameter (2) aus dem zweiten Messsignal (S2) zu erzeugen, der indikativ für einen Zeitpunkt (t_{gef}) eines Blutpulses in dem thorakalen Gewebe ist, der dem Blutauswurf zugeordnet ist;
- eine an der Überwachungsanordnung (20) angeschlossene Auswerteeinheit (30), die eingerichtet ist, eine Pulswellenlaufzeit (Δt) aus dem ersten Parameter (P1) und dem zweiten Parameter (P2) zu erzeugen;
wobei
die Detektoranordnung (10, 10', 10") eine Impedanzmesseinheit (12) mit einer Elektrodenanordnung (11, 11') umfasst, die eingerichtet sind, wenigstens das zweite Messsignal (S2) in Form eines Impedanzsignals (S) zu detektieren.

Unter einem Impedanzsignal ist vorliegend insbesondere ein Signal als Ergebnis einer Spannungsabtastung bei bekannter Stromaufprägung oder ein Signal des Ergebnisses einer Stromabtastung bei bekannter Spannungsaufprägung oder ein ähnliches Signal zu verstehen, mit dem auf die Impedanz zwischen zwei Polen der Elektrodenanordnung, nämlich das Impedanzsignal, geschlossen werden kann. Insbesondere betrifft dies die Impedanz bei einer bestimmten Frequenz eines elektrischen Stroms bzw. einer elektrischen Spannung. Die Impedanz kann auch aus einem pulsförmigen Wechselstrom bzw. einer pulsförmigen Wechselspannung ermittelt sein.

Unter einem Parameter ist vorliegend jedes quantifizierbare Merkmal eines Messsignals zu verstehen. Vorliegend ist ein Parameter insbesondere dann indikativ für einen Zeitpunkt, wenn er ein ausreichend zeitlich einschränkbares Merkmal des Messsignals definiert. Grundsätzlich kann der Parameter aus dem Messsignal selbst oder auch aus einem aus dem Messsignal z. B. durch Filterung oder mathematische Ableitung oder dergleichen gewonnenen Signal erzeugt werden. Ein Messsignal liegt insbesondere dann in Form eines Impedanzsignals vor, wenn es sich aus einem aufgrund einer Impedanzmessung gewonnenen Signal unmittelbar oder mittelbar ergibt.

Die Erfindung setzt bei der Überlegung an, dass es grundsätzlich möglich ist, einen für einen Zeitpunkt t_{sys} eines Blutauswurfs aus dem Herzen indikativen ersten Parameter aus einem dem kardialen Blutstrom zugeordneten Messsignal zu erzeugen. Die Erfindung schließt die Überlegung ein, dass sich ein erstes Messsignal in besonders vorteilhafter Weise als ein elektrisches Messsignal, beispielsweise als ein epithorakales Elektrokardiogramm realisieren lässt. Ausgehend von dieser Überlegung beruht die Erfindung auf der Erkenntnis, dass es für die Realisierung eines vergleichsweise einfach und dennoch verlässlich aufgebauten elektromedizinischen Implantats zur Bestimmung der Pulswellenlaufzeit von besonderem Vorteil sein kann, einen für den Zeitpunkt t_{gef} eines Blutpulses in dem thorakalen Gewebe indikativen zweiten Parameter aus einem zweiten elektrischen Messsignal zu erzeugen. Dieser Erkenntnis folgend wird vorgeschlagen, wenigstens das zweite Messsignal in Form eines Impedanzsignals zu detektieren und die Detektoranordnung mit einer entsprechend eingerichteten Impedanzmesseinheit und einer Elektrodenanordnung zu versehen.

Besonders vorteilhaft ist das elektromedizinische Implantat gemäß dem Konzept der Erfindung als ein reines Monitoringimplantat, d.h. ohne Therapieoption realisiert. Das Monitoringimplantat lässt sich vorteilhaft subkutan oder submuskulär implantieren und benötigt zur Überwachung der Pulswellenlaufzeit keine intrakardialen Elektroden. Insbesondere bietet das elektromedizinische Implantat die Möglichkeit, sowohl ein dem kardialen Blutstrom zugeordnetes Messsignal als auch ein dem epithorakalen peripheren Blutstrom zugeordnetes Messsignal besonders einfach und verlässlich zu erfassen. Das Implantat ist kompakt und einfach realisierbar.

In der Überwachungsanordnung lassen sich vorteilhaft an sich bekannte Maßnahmen nutzen, um aus einem Impedanzsignal einen zweiten Parameter zu erzeugen, der - als zweiter Parameter - indikativ für einen Zeitpunkt jenes Blutpulses in dem thorakalen Gewebe ist, welcher dem mit dem ersten Parameter erfassten Blutauswurf zugeordnet ist vorteilhaft zusätzlich, um aus einem Impedanzsignal einen ersten Parameter zu erzeugen, der - als erster Parameter - indikativ für den Blutauswurf ist.

Das dem kardialen Blutstrom zugeordnete erste Messsignal lässt sich im Rahmen des Konzepts der Erfindung auch auf vielfältige andere Art und Weise zur Verfügung stellen. In der Detektoranordnung können vorteilhaft an sich bekannte Maßnahmen genutzt werden, um aus dem ersten Messsignal den ersten Parameter zu erzeugen, der indikativ für den Zeitpunkt eines Blutauswurfs aus dem Herzen.

Die Auswerteeinheit ist ausgebildet, Verfahren auszuführen, um aus dem ersten und zweiten Parameter bzw. aus der Zeitdifferenz zwischen dem Blutauswurf aus dem Herzen und dem Zeitpunkt jenes Blutpulses in dem thorakalen Gewebe, welcher dem Blutauswurf zugeordnet ist, die Pulswellenlaufzeit zu bestimmen. Diese kann genutzt werden, um Aussagen über einen Gefäßzustand und/oder einen Blutdruck des Lebewesens mit dem elektromedizinischen Implantat zu machen.

Die Impedanzmesseinheit, die Überwachungsanordnung und die Auswerteeinheit sind vorteilhaft Teil einer in einem Gehäuse des Implantats untergebrachten elektronischen Schaltung, die wenigstens zur Abnahme des zweiten Messsignals mit der Elektrodenanordnung elektrisch verbunden ist. Die Elektrodenanordnung kann vorteilhaft entweder über einen sogenannten Header oder direkt am Gehäuse angebracht sein. Die elektronische Schaltung ist vorteilhaft als Mess- und Steuerelektronik ausgebildet. Das Gehäuse enthält vorzugsweise eine Batterie. Im Rahmen einer besonders bevorzugten Weiterbildung weist das Implantat ein Kommunikationsmodul zur drahtlosen Übermittlung der Pulswellenlaufzeit und/oder eines von der Auswerteeinheit auf Grundlage der Pulswellenlaufzeit gebildeten Auswerteergebnisses vom Implantat auf. Das Kommunikationsmodul kann vorzugsweise als ein Telemetriemodul im Gehäuse untergebracht sein. Eine telemetrische Anbindung des elektromedizinischen Implantats an das Überwachungssystem über das Kommunikationsmodul ermöglicht eine automatische Fernüberwachung des Lebewesens mittels des elektromedizinischen Implantats. Der Patient/das Lebewesen lässt sich somit durch den Arzt oder ein Servicecenter fernüberwachen, es können Alarmzustände aufgrund der übermittelten Pulswellenlaufzeiten und/oder Auswertesignale generiert werden, die im Servicecenter und/oder direkt beim Arzt angezeigt werden. So kann insbesondere ein Arzt über kritische Zustände eines Lebewesens/Patienten über das Servicecenter benachrichtigt werden, beispielsweise direkt oder mittels weiterer Kommunikationsmittel, wie einer Intemetplattform, einer SMS, einer Email, einem Fax oder Ähnlichem. Ein Überwachungssystem ist insbesondere in der Lage, durch das elektromedizinische Implantat, insbesondere in Form eines reinen Monitoringimplantats, angezeigte Patientenzustände für eine Visualisierung verfügbar zu machen.

Eine Verringerung der Pulswellenlaufzeit ist beispielsweise ein Indikator für eine verringerte Gefäßelastizität. Damit verbunden ist eine geringe Durchblutung der Koronargefäße und ein erhöhtes Infarktrisiko. Eine geringe Gefäßelastizität fördert die Verschlechterung des Gesundheitszustands bei chronischer Herzinsuffizienz. Die Gefäßelastizität beeinflusst auch die Therapiemöglichkeiten bei Hypertonie. Kenntnisse über die Gefäßelastizität und deren Veränderung können die Art und Dosierung von Medikamenten optimieren.

Auch der arterielle Blutdruck bzw. Änderungen des Blutdrucks können aus der Pulswellenlaufzeit geschätzt werden, so weit diese über einen ausreichenden Zeitraum bekannt ist. Der Blutdruck ist eine wichtige systematische Größe. Veränderungen des mittleren Blutdrucks, Änderungen des belastungsabhängigen Blutdruckanstiegs oder Blutdruckschwankungen können wichtige Hinweise auf die Entwicklung einer chronischen Krankheit, z.B. der arteriellen Hypertonie oder der Herzinsuffizienz geben. Solche und andere Auswerteergebnisse lassen sich vorteilhaft aus der Pulswellenlaufzeit bilden.

Zur Lösung der Aufgabe betreffend das Überwachungssystem führt die Erfindung auf ein Überwachungssystem aufweisend das elektromedizinische Implantat mit dem vorgenannten Kommunikationsmodul, wobei das Überwachungssystem weiter aufweist:
- eine Empfangseinheit zum Empfangen der Pulswellenlaufzeit und/oder des Auswerteergebnis;
- eine Speichereinheit für die Pulswellenlaufzeit und/oder das Auswerteergebnis.

Das Auswerteergebnis enthält insbesondere einen für den Blutgefäßzustand signifikanten Parameter und/oder einen für den Blutdruck signifikanten Parameter, die aus der Pulswellenlaufzeit bestimmt werden können. Außerdem kann das Auswerteergebnis weitere für einen Zustand des Lebewesens (Patienten) signifikante Parameter enthalten, die auf Grundlage der Pulswellenlaufzeit mit geeigneten Mitteln und Algorithmen grundsätzlich bestimmt werden können. Die Pulswellenlaufzeit und/oder das Auswerteergebnis werden über die Speichereinheit - die im Implantat oder bei der Empfangseinheit vorgesehen sein kann - für eine weitere Verwertung verfügbar gemacht. So können die Pulswellenlaufzeit und/oder das Auswerteergebnis in Form von Trends und Statistiken, z.B. Histogrammen, gespeichert und vom Arzt bei einer Nachsorgeuntersuchung mit einem geeigneten Abfragegerät abgefragt werden. Eine Abfrage kann aber auch vom Patienten selbst oder von einem autorisierten Servicecenter vorgenommen werden.

Das Konzept der Erfindung ermöglicht vorteilhaft, die durch das Implantat überwachte Pulswellenlaufzeit und die daraus bestimmbaren im Auswerteergebnis enthaltenen Parameter für eine weitere Analyse verfügbar zu machen. Dazu können geeignete weitere Verarbeitungseinheiten oder Abfrageeinheiten im Rahmen des Überwachungssystems vorgesehen werden, die vorzugsweise drahtlos mit dem Überwachungssystem und/oder dem Implantat, insbesondere mit der Empfangseinheit, kommunizieren können und/oder auf die Speichereinheit zugreifen können.

Erfindungsgemäß wird die eingangs genannte Aufgabe auch durch ein Verfahren zum Überwachen eines kardialen Blutstroms und eines epithorakalen peripheren Blutstroms eines Lebewesens gelöst, mittels
- eines elektromedizinischen epithorakal implantierbaren Implantats aufweisend die Schritte:
- Erfassen eines dem kardialen Blutstrom zugeordneten ersten Messsignals und eines dem epithorakalen peripheren Blutstrom zugeordneten zweiten Messsignals;
- Erzeugen eines ersten Parameters aus dem ersten Messsignal, der indikativ für einen Zeitpunkt t_{sys} eines Blutauswurfs aus dem Herzen ist, und
- Erzeugen eines zweiten Parameters aus dem zweiten Messsignal, der indikativ für einen Zeitpunkt t_{gef} eines Blutpulses in dem thorakalen Gewebe ist, der dem Blutauswurf zugeordnet ist;
- Erzeugen einer Pulswellenlaufzeit Δt aus dem ersten Parameter und dem zweiten Parameter, wobei das zweite Messsignal in Form eines Impedanzsignals detektiert wird.

Vorteilhaft kann im Überwachungssystem eine Triggereinheit zur zeitlich getriggerten Aktivierung des Kommunikationsmoduls, beispielsweise automatisch, ereignisgesteuert oder patienten- oder arztgesteuert, vorgesehen sein. Dadurch lässt sich eine ständige oder bedarfsmäßige Verfügbarkeit der Pulswellenlaufzeit und/oder des Auswertesignals für eine Weiterverarbeitung oder Analyse sicherstellen.

Vorteilhaft ist im Überwachungssystem eine Verarbeitungseinheit zur Verarbeitung bzw. Umwandlung der Pulswellenlaufzeit und/oder des Auswerteergebnisses visualisierende Datenform vorgesehen. Das Auswerteergebnis ermöglicht die Darstellung eines beispielsweise für einen kritischen Zustand eines Patienten indikativen Parameters. Eine visualisierende Darstellung des Auswerteergebnisses eignet sich beispielsweise, um einen kritischen Parameter besonders übersichtlich, z.B. in einem Histogramm oder in einer farblichen Darstellung zu visualisieren.

Das Überwachungssystem weist darüber hinaus vorteilhaft eine Abfrageeinheit zum Abfragen wenigstens der Pulswellenlaufzeit und/oder des Auswertesignals von dem elektromedizinischen Implantat und/oder der Speichereinheit auf. Die Abfrageeinheit kann Teil eines Servicecenters oder ein Patientengerät oder ein Arztgerät sein. Alle Beteiligten können an der Kommunikation mit dem Überwachungssystem und/oder dem Implantat selbst beteiligt werden. Die Empfangseinheit kann auch direkt einem Arzt oder einem Patienten oder einem Servicecenter zugeordnet sein.

Weitere vorteilhafte Weiterbildungen des Implantats der Erfindung sind den Unteransprüchen zu entnehmen und geben im Einzelnen vorteilhafte Möglichkeiten an, das oben erläuterte Konzept im Rahmen der Aufgabenstellung sowie hinsichtlich weiterer Vorteile zu realisieren.

Vorteilhaft bildet die Elektrodenanordnung eine Anzahl von Polen aus, über welche mittels der Impedanzmesseinheit eine Anzahl von Impedanzsignalen detektierbar sind. Einem Pol ist insbesondere jeweils eine Elektrode der Elektrodenanordnung zugeordnet, die zur Darstellung des Pols einen Elektrodenkörper aufweist. So lässt sich beispielsweise im Rahmen einer Zweipolmessung für ein Impedanzsignal eine Spannungsabtastung über die gleichen Elektroden realisieren, über welche auch der Strom aufgeprägt wird. Im Rahmen einer Dreipolmessung kann für ein Impedanzsignal auch eine Spannungsabtastung über eine erste Elektrode erfolgen, über welche ein Strom aufgeprägt wird als auch über eine zweite andere verfügbare Elektrode. Im Rahmen einer Vierpolmessung kann für ein Impedanzsignal eine Spannungsabtastung über mindestens zwei verfügbare Elektroden erfolgen, die nicht für eine Stromaufprägung genutzt werden. Grundsätzlich lässt sich eine Anzahl von Elektroden der Elektrodenanordnung einer Anzahl von Polen zuordnen, die für eine bevorzugte Messkonfiguration zweckmäßig ist. Unterschiedliche Messkonfigurationen können auch in beliebiger Kombination gleichzeitig oder alternierend vorgesehen sein. Dadurch können vorteilhaft mehrere Impedanzsignale mit unterschiedlichem Informationsgehalt wenigstens für das zweite Messsignal zur Verfügung gestellt werden.

Nach einer ersten besonders bevorzugten Weiterbildung ist vorgesehen, dass die Impedanzmesseinheit und die Elektrodenanordnung eingerichtet sind, das erste Messsignal in Form eines Impedanzsignals zu detektieren. Vorteilhaft lässt sich gemäß einer solchen Weiterbildung das erste und das zweite Messsignal mit einer einzigen entsprechend ausgelegten Elektrodenanordnung in Form eines Impedanzsignals realisieren.

Insbesondere hat es sich als vorteilhaft erwiesen, dass die Elektrodenanordnung wenigstens eine vergleichsweise nahe zum Implantat angeordnete Elektrode umfasst. Die nahe zum Implantat angeordnete Elektrode kann separat vom Gehäuse, unmittelbar auf dem Gehäuse oder von dem Gehäuse des Implantats selbst gebildet sein. Es hat sich gezeigt, dass mit nahe zum Implantat liegenden Elektroden vorrangig Effekte in der unmittelbaren Umgebung des Implantats erfasst werden können. Es hat sich gezeigt, dass sich eine implantatsnahe Elektrode besonders bevorzugt eignet, das zweite Messsignal in Form des Impedanzsignals mit zu detektieren. Insbesondere kann die Elektrodenanordnung auch wenigstens eine entfernt zum Implantat angeordnete weitere Elektrode aufweisen. Implantatfern angeordnete weitere Elektroden haben sich als besonders geeignet erwiesen, Effekte in größerer Entfernung vom Implantat zu erfassen. Insbesondere eignen sie sich in vorteilhafter Weise zur Detektion des ersten Messsignals in Form eines Impedanzsignals. Eine implantatnahe Elektrode hat einen geringeren Abstand zum Implantat als eine implantatferne Elektrode. Beispielsweise lässt sich eine Elektrodenanordnung derart ausbilden, dass eine implantatnahe Elektrode, insbesondere dessen Elektrodenkörper und/oder Pol, näher am Implantat angeordnet ist als eine implantatferne Elektrode, insbesondere dessen Elektrodenkörper und/oder Pol.

In vorteilhafter Weise weist die Impedanzmesseinheit ein Erregermodul auf, das zum Abgeben eines elektrischen Stroms über die Elektrodenanordnung und ein Tastmodul das zum Annehmen einer Spannung über die Elektrodenanordnung ausgebildet ist. Umgekehrt kann das Erregermodul auch zum Abgeben einer Spannung über die Elektrodenanordnung und ein Tastmodul zum Annehmen eines Stroms über die Elektrodenanordnung genutzt werden. Vorteilhaft kann ein Erreger- und Abtastmodul in einem gemeinsamen Modul - im Folgenden auch als Frequenzmodulator bezeichnet - realisiert sein. Durch Abgabe eines Stroms oder einer Spannung wird diese dem anliegenden Gewebe aufgeprägt. Umgekehrt kann eine vom umliegenden Gewebe getragene Spannung oder Strom abgetastet werden.

Im Rahmen einer weiteren besonders bevorzugten ersten Variante hat es sich als vorteilhaft erwiesen, dass das zweite Messsignal in Form eines zweiten Impedanzsignals auf Basis einer zweiten Erregerfrequenz gebildet ist. Zusätzlich oder alternativ hat es sich bei dieser Variante als besonders vorteilhaft erwiesen, dass das erste Messsignal in Form eines ersten Impedanzsignals auf Basis einer ersten Erregerfrequenz gebildet ist und ein oder mehrere Erregermodule ausgebildet sind. Das erste Messsignal und das zweite Messsignal lassen sich vorteilhaft als ein einziges Impedanzsignal zur Verfügung stellen und über ein oder mehrere Abtastmodule als zwei unterschiedliche Frequenzanteile über dessen Frequenz, beispielsweise unter Nutzung eines Frequenzfilters, aus dem Impedanzsignal bestimmen.

Darüber hinaus hat es sich als vorteilhaft erwiesen, dass die zweite Erregerfrequenz für das zweite Messsignal niedriger ist, als die erste Erregerfrequenz für das erste Messsignal. Es hat sich gezeigt, dass bei einer vergleichsweise hohen Erregerfrequenz ein zeitlicher Impedanzverlauf messbar ist, bei dem insbesondere die Volumenänderung des Herzens gut erfassbar wird. Nach geeigneter Frequenz-Hochpass-Filterung kann aus einem solchen Impedanzverlauf mit höherer Erregerfrequenz besonders vorteilhaft der Zeitpunkt eines Blutauswurfs aus dem Herzen bestimmt werden. Vorliegend kann dazu insbesondere der Zeitpunkt eines Beginns einer Ventrikelkontraktion t_{sys} bestimmt werden.

Es hat sich auch gezeigt, dass bei vergleichsweise niedriger Erregerfrequenz besonders vorteilhaft ein zeitlicher Impedanzverlauf messbar ist, mit dem die Durchblutung des das Implantat umgebenden Gewebes erfassbar ist, d.h. ein zweites Messsignal erzeugbar ist, das indikativ einen Zeitpunkt eines Blutpulses in dem thorakalen Gewebe ist. Nach geeigneter Frequenz-Tiefpass-Filterung kann aus einem solchen Impedanzverlauf der Zeitpunkt eines Eintreffens einer Blutdruckwelle in einem thorakalen Gefäß t_{gef} bestimmt werden.

Eine das erfindungsgemäße Konzept in besonders vorteilhafter Weise weiterbildende Kombination sieht entsprechend vor, dass das zweite Messsignal in Form eines zweiten Impedanzsignals auf Basis einer zweiten Erregerfrequenz und mit einer implantatnahen Elektrode der Elektrodenanordnung gebildet ist und das erste Messsignal in Form eines ersten Impedanzsignals auf Basis einer ersten Erregerfrequenz mit einer implantatfernen distalen Elektrode der Elektrodenanordnung gebildet ist, wobei die zweite Erregerfrequenz niedriger als die erste Erregerfrequenz ist.

Insbesondere hat es sich zur Weiterbildung als vorteilhaft erwiesen, dass die Detektoreinheit ein Zeittaktmodul umfasst, welches ausgebildet ist, das zweite Messsignal und das erste Messsignal zeitlich zu sequenzieren und/oder zu alternieren. Vorteilhaft kann ein dem zweiten Messsignal zugeordnetes Impedanzsignal zeitlich nach oder vor einem dem ersten Messsignal zugeordneten Impedanzsignal detektiert werden. Die Abfolge vom ersten und zweiten Messsignal lässt sich auch alternierend mehrfach wiederholen. Vorteilhaft eignet sich dazu ein Multiplexer oder dergleichen.

Im Rahmen einer bevorzugten Weiterbildung weist die Detektoreinheit einen Frequenzmodulator und/oder Detektor auf, mit dem die obigen Funktionen realisierbar sind. Besonders bevorzugt kann über einen Frequenzmodulator ein Hintergrundsignal in Form eines hochfrequenten Impedanzmesssignals ermittelbar sein. Die Erregerfrequenz eines hochfrequenten Hintergrund-Impedanzmesssignals liegt insbesondere über der obigen ersten und/oder zweiten Erregerfrequenz. Es hat sich überraschend herausgestellt, dass eine Veränderung der Frequenz für eine Impedanzmessung zu einem veränderten Kontrast zwischen Blut und Gewebe (Haut und Muskeln) führt. Die Weiterbildung hat erkannt, dass Blut eine höhere Leitfähigkeit als das Gewebe hat und dieser Abstand mit zunehmender Frequenz abnimmt, so dass bei niedrigeren Frequenzen ein höherer Kontrast zwischen Blut und Gewebe besteht als auch bei hohen Frequenzen. Die Weiterbildung nutzt diesen Ef fekt, um rein geometrische Änderungen im Rahmen eines Hintergrundsignals in Form eines hochfrequenten Hintergrund-Impedanzsignals zu ermitteln und geometrische Änderungen in einem ersten und/oder zweiten Messsignal zu unterdrücken, indem das erste und/oder zweite Messsignal in Relation zum Hintergrund-Impedanzsignal gesetzt wird.

Im Rahmen einer besonders bevorzugten weiterbildenden zweiten Variante ist vorgesehen, dass das zweite Messsignal ein über eine Amplitudenschwelle separierbares Teilsegment eines transienten Impedanzmesssignals ist. Vorteilhaft ist auch das erste Messsignal als ein über eine Amplitudenschwelle separierbares Teilsegment eines transienten Impedanzmesssignals. Im Rahmen einer vorteilhaften Realisierung dieser zweiten Variante kann dazu vorgesehen sein, dass das Teilsegment des ersten Messsignals zur Bestimmung des Zeitpunkts eines Blutauswurfs (der Systole) t_{sys} mit einem geeigneten Algorithmus bestimmt wird. In dem anderen Teilsegment des transienten Impedanzsignals kann bei einer vorteilhaften Realisierung dieser zweiten Variante der Zeitpunkt t_{gef} eines Blutpulses in dem thorakalen Gewebe mit einem anderen entsprechend geeigneten Algorithmus bestimmt werden.

Mit Vorteil versehen weist die Elektrodenanordnung eine Elektrode auf, die symmetrisch um einen Gehäusebehälter des Implantats angeordnet ist. Insbesondere hat es sich dazu als vorteilhaft erwiesen, dass die Elektrode mehrseitig oder allseitig oder ringförmig um einen Gehäusebehälter des Implantats angeordnet ist. Es hat sich gezeigt, dass sich mittels einer symmetrisch um einen Gehäusebehälter des Implantats angeordneten Elektrode die Elektrodenanordnung so ausbilden lässt, dass wenigstens das zweite Messsignal in Form eines Impedanzsignals unabhängig von einer Winkellage des Implantats im Körpergewebe ermittelbar ist. Vorteilhaft bildet der Gehäusebehälter selbst eine Elektrode aus.

Im Rahmen einer besonders vorteilhaften zweiten Weiterbildung ist vorgesehen, dass die Detektoranordnung zum Erfassen des ersten Messsignals eine EKG-Messeinheit mit einer Elektrodenanordnung umfasst, die eingerichtet sind, das erste Messsignal in Form eines Elektrokardiogrammsignals (EKG) zu detektieren. Diese Form des ersten Messsignals hat sich als besonders verlässlich erwiesen.

Darüber hinaus können im Rahmen einer dritten besonders vorteilhaften Weiterbildung zusätzlich oder alternativ zum ersten Messsignal in Form eines Impedanzsignals weitere Detektoren vorgesehen sein, das erste Messsignal zu detektieren. Als besonders bevorzugt hat sich ein akustischer Sensor erwiesen, mit dem Herztöne detektierbar sind, so dass daraus Klappenverschlusszeiten bestimmbar sind. Als vorteilhaft hat sich auch ein Beschleunigungssensor erwiesen, mit dem ein Blutauswurf direkt detektierbar ist. Ein bereits erläuterter Impedanzsensor zur Detektion des ersten Messsignals in Form eines Impedanzsignals bestimmt besonders vorteilhaft den Zeitpunkt des Blutauswurfs aus dem Ventrikel.

Vorteilhaft umfasst die Detektoranordnung zum Erfassen des zweiten Messsignals einen oder mehrere Detektoren, die ausgewählt sind aus der Gruppe bestehend aus: akustischem Detektor, optischem Detektor, Druck- und/oder Dehnungsdetektor, Beschleunigungsdetektor. Solche und andere Detektoren können vorteilhaft eingesetzt werden, zusätzlich zur Elektrodenanordnung und der Impedanzmesseinheit zur Detektion des zweiten Messsignals in Form eines Impedanzsignals. So kann in vorteilhafter Weise ein akustischer Detektor genutzt werden, um das Strömungsgeräusch eines epithorakalen peripheren Blutstroms im Gefäß zu detektieren. Der optische Detektor kann vorteilhaft zur Erfassung eines Plethysmogramms für den Blutstrom genutzt werden. Ein mechanischer Dehnungs- oder Kompressionssensor oder sonstiger Drucksensor kann vorteilhaft für die Erfassung eines Volumenpulses in angrenzenden Blutgefäßen, d.h. zur Messung eines epithorakalen peripheren Blutstroms genutzt werden. Ein Beschleunigungsdetektor kann die durch einen Volumenpuls hervorgerufene Bewegung des Gehäuses oder eines über ein Kabel abgesetzten Detektors detektieren. Besonders vorteilhaft kann der akustische Detektor als Durchflussdetektor ausgelegt sein, welcher die Blutflussgeschwindigkeit in der Umgebung des Implantats für einen epithorakalen peripheren Blutstrom detektiert. Der akustische Durchflussdetektor ist vorteilhaft auf Basis von Ultraschall realisiert. Ein Hochfrequenzdetektor kann vorteilhaft genutzt werden, um die Änderung von dielektrischen Eigenschaften eines das Implantat umgebenden Gewebes durch die Pulswelle im epithorakalen peripheren Blutstrom zu detektieren. Alle vorgenannten Detektoren eignen sich, um zusätzlich ein dem epithorakalen peripheren Blutstrom zugeordnetes zweites Messsignal zu generieren und daraus einen zweiten Parameter zu erzeugen, welcher indikativ für einen Zeitpunkt t_{gef} eines Blutpulses in dem thorakalen Gewebe ist und der dem Blutauswurf zugeordnet ist.

Zusammenfassend sehen die vorgenannten Weiterbildungen zusätzlich zur Impedanzmesseinheit in der Detektoranordnung bei Bedarf weitere Detektoren vor, die es erlauben, zusätzlich zum zweiten Messsignal in Form eines Impedanzsignals weitere zweite Messsignale zu generieren, aus denen ein zweiter Parameter erzeugt und überprüft wird, der indikativ einen Zeitpunkt t_{gef} eines Blutpulses in dem thorakalen Gewebe ist. Des Weiteren kann zusätzlich oder alternativ zur Erzeugung eines ersten Messsignals in Form eines Impedanzsignals eine der vorgenannten Detektoreinheiten genutzt werden, um das erste Messsignal oder ein weiteres erstes Messsignal zu generieren, aus dem ein erster Parameter erzeugbar ist, der indikativ einen Zeitpunkt t_{sys} eines Blutauswurfs aus dem Herzen ist.

Ausführungsbeispiele der Erfindung werden nun nachfolgend anhand der Zeichnung beschrieben. Diese soll die Ausführungsbeispiele nicht notwendigerweise maßstäblich darstellen, vielmehr ist die Zeichnung, wo zur Erläuterung dienlich, in schematisierter und/oder leicht verzerrter Form ausgeführt. Im Hinblick auf Ergänzungen der aus der Zeichnung unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausfiihrungsform vorgenommen werden können, ohne von der allgemeinen Idee der Erfindung abzuweichen. Die in der Beschreibung, in der Zeichnung sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, der Zeichnung und/oder den Ansprüchen offenbarten Merkmale. Die allgemeine Idee der Erfindung ist nicht beschränkt auf die exakte Form oder das Detail der im folgenden gezeigten und beschriebenen bevorzugten Ausführungsform oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsbeispiele sowie anhand der Zeichnung; diese zeigt in:
- Fig. 1: eine symbolische Darstellung eines Überwachungssystems gemäß einer bevorzugten Ausführungsform der Erfindung;
- Fig. 2: eine erste bevorzugte Ausfiihrungsform eines Implantats in Form eines Monitoringimplantats zum Überwachen eines kardialen Blutstroms und eines epithorakalen peripheren Blutstroms eines Lebewesens zur Verwendung im Überwachungssystem der Fig. 1,
- Fig. 3A, 3B: eine erste frequenzbasierte und zweite amplitudenbasierte Alternative zur Realisierung zweier Impedanzsignale und einer Auswertung derselben bei einem elektromedizinischen Implantat der Fig. 2;
- Fig. 4: ein elektromedizinisches Implantat in Form eines Monitoringimplantats gemäß einer zweiten bevorzugten Ausführungsform, bei welcher das erste Messsignal in Form eines EKG-Signals gebildet ist;
- Fig. 5: eine dritte bevorzugte Ausführungsform eines elektromedizinischen Implantats in Form eines Monitoringimplantats, bei welcher das erste Messsignal in Form eines akustischen Signals gebildet ist.
- Fig. 6: eine vierte bevorzugte Ausführungsform eines elektromedizinischen Implantats in Form eines Monitoringimplantats mit einer alternativen besonders vorteilhaften Elektrodenanordnung.

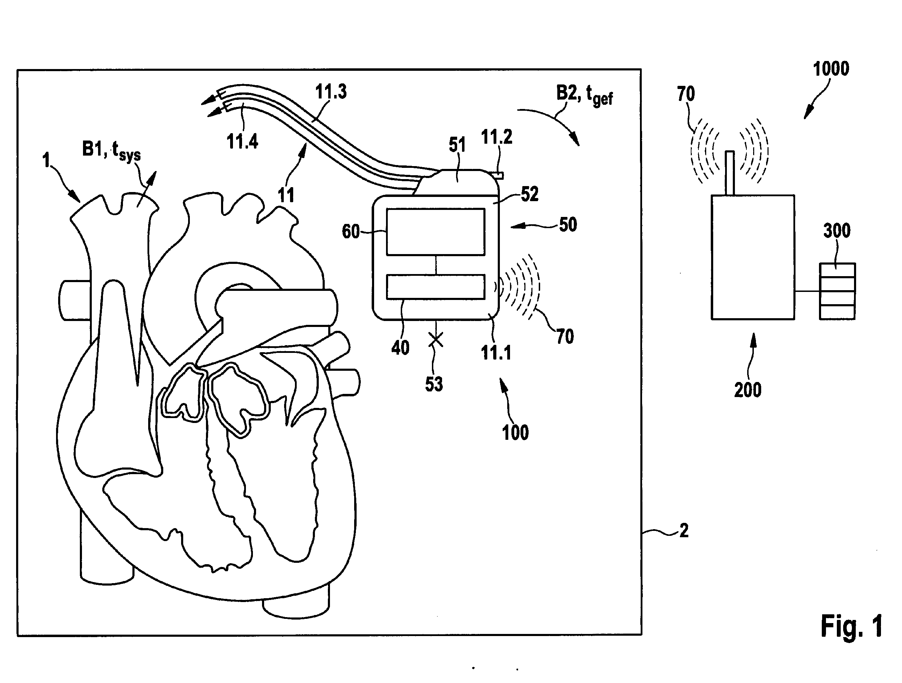

Fig. 1 zeigt eine symbolische Darstellung einer bevorzugten Ausführungsform eines Überwachungssystems 1000 mit einem elektromedizinischen Implantat 100, das zum Überwachen eines ebenfalls symbolisch dargestellten kardialen Blutstroms B1 und eines epithorakalen peripheren Blutstroms B2 eines Lebewesens, nämlich regelmäßig eines Patienten mit oder ohne Indikation auf eine Herzinsuffizienz, ausgelegt ist. Dazu ist das Herz 1 des Patienten und ein Thorax 2 des Patienten zusammen mit den vorgenannten Blutströmen B1 und B2 symbolisch dargestellt. Das Implantat 100 ist vorliegend als reines Monitoringimplantat, d.h. ohne Therapiefunktion ausgelegt. Das Implantat 100 hat ein Gehäuse 50 mit einem Gehäusebehälter 52 und einem sogenannten Header 51 sowie eine am Gehäuse 50 angeschlossene Elektrodenanordnung 11. Das Implantat 100 selbst ist in dem Thorax 2 des Patienten subkutan im Gewebe des Patienten angeordnet. Um eine vergleichsweise gute Lagestabilisierung des Implantats 100 zu erreichen und insbesondere eine Migration des Implantats sowie Bewegungsartefakte des Patienten auf Überwachungssignale zu vermeiden, ist am Gehäuse 50 des Implantats ein Verankerungsmittel 53 vorgesehen, welches das Implantat 100 im subkutanen Gewebe verankert. Ein derartiges Verankerungsmittel kann z.B. als Einnähöse, als Ankerstruktur, Schraube oder dergleichen ausgefiihrt sein. Vorliegend ist das Verankerungsmittel 53 reversibel ausgeführt, so dass es leicht wieder vom Gewebe des Patienten lösbar ist - das Implantat 100 kann somit bei Bedarf aus dem Thorax 2 des Patienten entfernt werden.

Die Elektrodenanordnung 11 weist als Elektroden zunächst eine durch den Gehäusebehälter 52 gebildete erste Elektrode 11.1, eine am Header 51 angeordnete zweite Elektrode 11.2 und zwei über Kabel abgesetzte Elektroden 11.3, 11.4 auf. In dem Gehäusebehälter 52 ist eine Elektronik 60 mit daran angeschlossenem Kommunikationsmodul 40 angeordnet. Die vorliegend als Mess- und Steuerelektronik ausgelegte Elektronik 60 weist eine im Folgenden erläuterte Detektoranordnung 10, Überwachungsanordnung 20 und Auswerteeinheit 30 auf. Das Kommunikationsmodul 40 weist eine Antenne oder dergleichen Telemetrieeinheit zur Darstellung einer drahtlosen Telemetrieverbindung 70 mit einer außerhalb des Patienten angeordneten Empfangseinheit 200 auf. Die Empfangseinheit 200 dient als Teil des Überwachungssystems 1000 der Patientenfernüberwachung durch einen Arzt oder ein Servicecenter. Weiter weist das Überwachungssystem 1000 eine Speichereinheit 300 auf, die mit der Empfangseinheit 200 über eine drahtgebundene oder drahtlose Schnittstelle verbunden ist und welche in der Lage ist, von dem Implantat 100 übermittelte Signalinhalte zu speichern. Ein Signalinhalt enthält eine sogenannte Pulswellenlaufzeit, welche sich aus der Überwachung des kardialen Blutstroms B1 und des epithorakalen peripheren Blutstroms B2 ergibt. Durch die Verfügbarkeit der Pulswellenlaufzeit Δt im Überwachungssystem 1000 bzw. auf dem Speicher 300 können auf Grundlage der Pulswellenlaufzeit gebildete Auswerteergebnisse und daraus abgeleitete Patientenwerte und/oder Patientenzustände, insbesondere kritische Zustände des Patienten, einem Arzt, dem Patienten selbst oder einem Servicecenter verfügbar gemacht werden. Dazu eignet sich beispielsweise eine nicht näher dargestellte Internetplattform oder eine sonstige nachrichtentechnische Anbindung des Überwachungssystems 1000 an ein weiteres breiter verfügbares Kommunikationssystem. Ein solches Kommunikationssystem kann neben einem Internetnetzwerk auch ein mobiles Kommunikationssystem, z.B. zur Übermittlung von SMS, E-Mail, Fax oder Ähnlichem sein. Das so angebundene Überwachungssystem 1000 ermöglicht es somit, insbesondere Alarmzustände aufgrund der vom Implantat 100 übermittelten Signalinhalte an einem Ort extern vom Implantat zu generieren. Dazu kann dem Patienten bereits bei Ankündigung eines kritischen Zustandes eine Hilfestellung oder Gegenmaßnahme vermittelt werden.

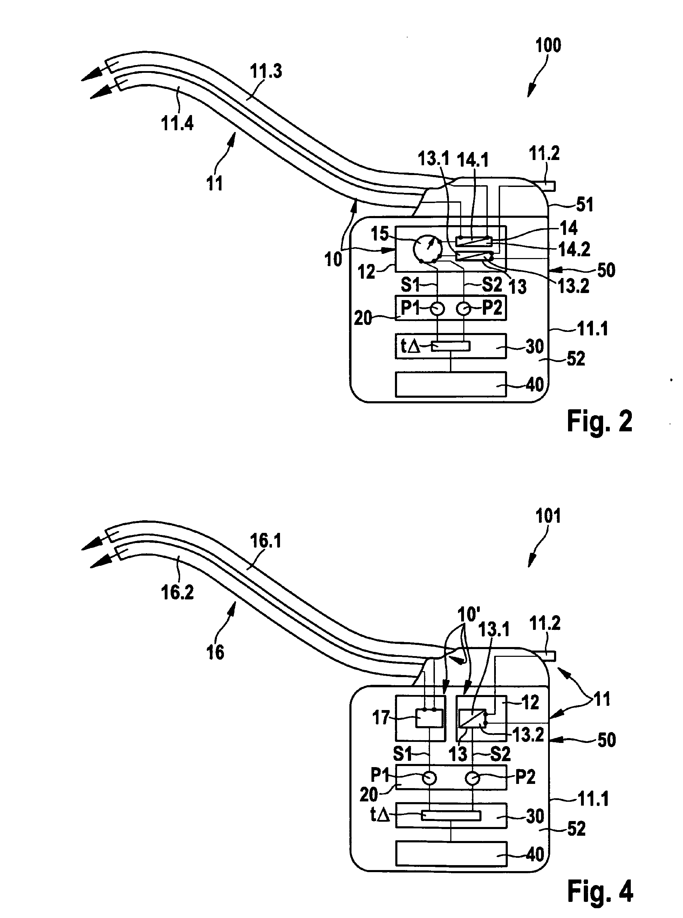

Die folgende Beschreibung der Fig. 2 bis Fig. 6 erläutert bevorzugte Ausführungsformen und Funktionen des Implantats 100 selbst. Dabei werden, wo zweckmäßig, für identische oder ähnliche Teile oder Teile identischer oder ähnlicher Funktion gleiche Bezugszeichen verwendet.

Fig. 2 zeigt in einer vergrößerten symbolischen Darstellung das elektromedizinische Implantat 100 des Überwachungssystems 1000 der Fig. 1. Das als Monitoringimplantat ausgebildete Implantat 100 weist eine Detektoranordnung 10 zum Erfassen eines dem kardialen Blutstrom B1 zugeordneten ersten Messsignals S1 und eines dem epithorakalen peripheren Blutstrom B2 zugeordneten zweiten Messsignals S2 auf. Teil der Detektoranordnung 10 sind die anhand von Fig. 1 erläuterten Elektroden 11.1, 11.2, 11.3, 11.4 der Elektrodenanordnung 11 sowie eine Impedanzmesseinheit 12.

Die Impedanzmesseinheit 12 sowie die an die Detektoranordnung 10 angeschlossene Überwachungsanordnung 20 und Auswerteeinheit 30 sind Teil der in Fig. 1 beschriebenen Mess- und Steuerelektronik 60. Die Überwachungsanordnung 20 ist eingerichtet, einen ersten Parameter P1 aus dem ersten Messsignal S1 zu erzeugen, der indikativ für einen Zeitpunkt t_{sys} eines Blutauswurfs aus dem Herzen ist. Des Weiteren ist die Überwachungsanordnung 20 eingerichtet, einen zweiten Parameter P2 aus dem zweiten Messsignal S2 zu erzeugen, der indikativ für einen Zeitpunkt t_{gef} eines Blutpulses in dem thorakalen Gewebe ist. Dies kann durch an sich bekannte Signalanalyseverfahren erfolgen. Der Blutpuls in dem thorakalen Gewebe lässt sich also durch Überwachen des epithorakalen peripheren Blutstroms B2 ermitteln, wobei der Blutpuls in dem thorakalen Gewebe dem Blutauswurf aus dem Herzen zugeordnet ist und der letztere durch Überwachen des kardialen Blutstroms B 1 ermittelbar ist.

Eine an der Überwachungsanordnung 20 angeschlossene Auswerteeinheit 30 ist eingerichtet, eine Pulswellenlaufzeit aus dem ersten Parameter P1 und dem zweiten Parameter P2 zu erzeugen. Vorliegend erfolgt dies dadurch, dass aus dem ersten Parameter P1 auf den Zeitpunkt t_{sys} eines Blutauswurfs aus dem Herzen geschlossen wird und von dem zweiten Parameter P2 auf den Zeitpunkt des Blutpulses in dem thorakalen Gewebe geschlossen wird. Dies kann durch an sich bekannte Signalauswertealgorithmen erfolgen. Vorliegend durch Subtrahieren von t_{gef} und t_{sys} ergibt sich eine Zeitdifferenz, welche als Pulswellenlaufzeit Δt=t_{gef}-t_{sys} bezeichnet wird. Die Umwandlung der Parameter P1, P2 in Zeitpunkte t_{gef}, t_{sys} sowie die Differenzbildung zu Δt erfolgt mittels der Auswerteeinheit 30. Weitere aufgrund der Pulswellenlaufzeit ermittelbare Auswerteergebnisse werden von der Auswerteeinheit 30 vorliegend nicht vorgenommen. Vielmehr wird die Pulswellenlaufzeit Δt an das Kommunikationsmodul 40 weitergegeben und von diesem durch Aufbau einer Telemetrieverbindung 70 an die Empfangseinheit 200 des Überwachungssystems 1000 übermittelt und in der Speichereinheit 300 desselben verfügbar gemacht. Damit steht die Pulswellenlaufzeit Δt dem Überwachungssystem 1000 zur Verfügung, und kann vom Überwachungssystem, beispielsweise durch eine nicht näher erläuterte Prozessoreinheit, weiterverarbeitet werden, z.B. zu weiteren Auswerteergebnissen und Patientenwerten, die indikativ für einen Patientenzustand sind. Solche Auswerteergebnisse und Patientenwerte können auch vorteilhaft visualisiert werden, beispielsweise im Rahmen von Histogrammen, transienten Signalen oder Mittelwerten, um einem Arzt oder einem Servicecenter auf möglichst intuitive und schnell erkennbare Weise kritische Zustände des Patienten zu verdeutlichen. Auf diese Weise können Alarmzustände einem Arzt, dem Patienten oder dem Servicecenter selbst verfügbar gemacht werden, indem der Arzt, der Patient oder das Servicecenter Zugriff auf den Speicher 300 des Überwachungssystems 1000 hat.

Im Einzelnen kann das Implantat 100 im Rahmen einer Auswertung über das Überwachungssystem 1000 für ein so genanntes Home-Monitoring genutzt werden. Beispielsweise können Messdaten und sonstige Auswerteergebnisse, basierend auf der Pulswellenlaufzeit sowie die Pulswellenlaufzeit selbst, wie erläutert, in einer Speichereinheit 300 des Überwachungssystems 1000 extern vom Implantat 100 oder auch im Implantat 100 gespeichert sein. Dazu können die Auswerteergebnisse und/oder die Pulswellenlaufzeit in Form von Trends, Statistiken und dergleichen, z.B. als Histogramme, einem Arzt, Servicecenter oder Patienten zur Verfügung gestellt werden oder auf Wunsch interrogiert werden. So kann beispielsweise ein Arzt oder ein Servicecenter oder ein Patient mit einer geeigneten Abfrage - beispielsweise mit einem Abfragegerät, welches die Empfangseinheit 200 umfasst - interrogiert werden und so für eine Nachsorgeuntersuchung zur Verfügung stehen.

Auch können Auswerteergebnisse und/oder die Pulswellenlaufzeit selbst in regelmäßigen Zeitabständen, z.B. einmal pro Tag, an die Empfangseinheit 200 - wiederum als Teil eines Patienten-, Arzt- oder Servicecentergeräts - gesendet werden und von dort über eine geeignete Datenverbindung des Überwachungssystems 1000, also z.B. über eine drahtgebundene oder drahtlose Schnittstelle weitergegeben werden.

Eine solche Datenübertragung kann vom Patienten, vom Arzt oder auf sonstige Weise automatisch oder ereignisgesteuert oder personengesteuert getriggert werden. Eine personengetriggerte Auslösung einer Datenübertragung kann beispielsweise besonders einfach erfolgen, indem ein Magnet oder ein geeignetes Handgerät auf die Körperstelle aufgelegt wird, an der sich das Implantat unter der Haut befindet. Auf diese oder andere Weise kann ein Triggerimpuls an das Implantat 100 übermittelt werden, welcher das Kommunikationsmodul 40 aktiviert. Eine dann aufgebaute Telemetrieverbindung 70 ist in der Lage, die Pulswellenlaufzeit oder Auswerteergebnisse an die Empfangseinheit 200 zu übermitteln. Eine ereignisgesteuerte Triggerung kann beispielsweise durch Überschreiten eines Grenzwertes für die Pulswellenlaufzeit und/oder für eines der Auswerteergebnisse ausgelöst werden.

Eine Analyse kann, z.B. im Servicecenter, automatisch erfolgen, dabei können bedrohliche Zustände, eine Verschlechterung des Gesundheitszustandes, etc. diagnostiziert und visualisiert werden. Ein Arzt kann so besonders effektiv individuelle Patientenwerte erfassen und im Übrigen Details, beispielsweise in Form vorbereiteter Masken einsehen. Beispielsweise könnte ein Arzt oder eine andere Person über ein vorgenanntes externes Netzwerk, z.B. ein Internetnetzwerk oder ein mobiles Netzwerk auf die Speichereinheit 300 des Überwachungssystems 1000 zugreifen.

Die Darstellung von Daten der Pulswellenlaufzeit sowie sonstige Auswerteergebnisse umfasst insbesondere zeitliche Verläufe oder Kombinationen von mehreren Datenkanälen und extrahierten Signalmerkmalen. Dadurch kann ein umfassendes Bild eines aktuellen Patientenzustandes, also Gesundheitszustandes bzw. dessen zeitliche Veränderung bei einem Patienten dargestellt werden. Insbesondere lassen sich verschiedene Komponenten eines zeitlichen Verlaufs nach Änderungsintervallen separieren und für eine gewünschte Anwendung auswählen. Dies betrifft beispielsweise langfristige Änderungen, z.B. über einige Monate, um beispielsweise altersbedingte Änderungen einer Gefäßsteifigkeit zuzuordnen. Kurzfristige Änderungen mit Intervallen bis zu einem Tag können dabei physiologische Blutdruckschwankungen, wie einen Tag-Nacht-Rhythmus, eine Belastung, etc. zugeordnet werden. Verbleibende mittelfristige Änderungen, die sich zwischen mehreren Tagen bis hin zu mehreren Monaten erstrecken können, erlauben die Entwicklung einer Krankheit oder die Wirkung einer Therapie sichtbar zu machen. So können z.B. Blutdruckänderungen durch Trainingseffekte oder Diäteffekte sichtbar gemacht werden. Ebenso können Änderungen der Gefäßsteifigkeit durch Diabetes oder eine Entwicklung von chronischem Bluthochdruck oder Medikamentenwirkung dargestellt werden.

Anhand der Pulswellenlaufzeit Δt werden vorliegend vor allem diagnostische Aussagen über Patientenwerte, wie der Gefäßzustand und der Blutdruck des Patienten, unter Berücksichtigung von weiteren Auswerteergebnissen getroffen. Ein Patientenzustand, wie ein Herz-Kreislauf-Zustand des Patienten ist so verfügbar.

Die Standardauswertung für eine z.B. tägliche Blutdrucküberwachung umfasst beispielsweise einen täglichen Mittelwert, einen täglichen Minimalwert und einen täglichen Maximalwert des Blutdrucks.

Eine erweiterte Auswertung kurzfristiger Änderungen der Pulswellenlaufzeit zur Darstellung von Blutdruckänderungen, d.h. innerhalb eines Tages, eröffnet weitere diagnostische Möglichkeiten. Eine Visualisierung als Histogramm oder als Darstellung eines Tag-Nacht-Rhythmus des Blutdrucks, insbesondere mit Amplitudenphase oder einem 24-Stunden-Trend hilft dabei. Es kann auch in vorteilhafter Weise ein Zusammenhang von Blutdruck und Herzfrequenz dargestellt werden oder ein Zusammenhang von Blutdruck und Patientenbelastung. Eine Patientenbelastung kann beispielsweise mit einem im Implantat 100 zusätzlich untergebrachten Beschleunigungssensor ermittelbar sein. Relevant kann auch eine Blutdruckvariabilität sein, die gegebenenfalls aus einer Frequenzanalyse von Impedanzsignalen ermittelbar ist. Auch kann als Teil von weiteren Patientenwerten eine Baroreflex-Sensitivität oder -Latenzzeit ermittelbar sein. Diese ergeben sich beispielsweise aus dem Zusammenhang von dynamischem Blutdruck und Herzfrequenzänderungen.

In einer hier nicht dargestellten Ausfiihrungsform kann das vorliegend als reines Monitoringimplantat ausgeführte Implantat 100 auch als Therapieimplantat beispielsweise als CRM-Implantat, Herzschrittmacher oder Defibrillator ausgeführt sein.

Die weiteren anhand von Fig. 2 bis Fig. 6 ausgeführten Erläuterungen des Implantats 100 betreffen vor allem das Erfassen des dem kardialen Blutstrom B1 zugeordneten ersten Messsignals S1 um einen Zeitpunkt t_{sys} eines Blutauswurfs aus dem Herzen zu ermitteln und zum Erfassen eines dem epithorakalen peripheren Blutstrom B2 zugeordneten zweiten Messsignals S2 um einen Zeitpunkt t_{gef} eines Blutpulses in dem thorakalen Gewebe zu ermitteln.

Fig. 2 zeigt eine erste bevorzugte Ausfiihrungsform eines Implantats 100 in Form eines Monitoringimplantats zum Überwachen eines kardialen Blutstroms B 1 und eines epithorakalen peripheren Blutstroms B2.

Die Detektoranordnung 10 ist zum Erfassen eines dem Blutstrom B1 zugeordneten ersten Messsignals S 1 und eines dem Blutstrom B2 zugeordneten zweiten Messsignals S2 ausgelegt. Die Detektoranordnung 10 umfasst dazu eine Impedanzmesseinheit 12, die mit einer Elektrodenanordnung 11 verbunden ist.

Die Impedanzmesseinheit 12 weist einen ersten mit den Elektroden 11.1 und 11.2 verbundenen Frequenzmodulator 13 auf. Der Frequenzmodulator 13 umfasst ein Erregermodul 13.1 zum Aufprägen eines elektrischen Stroms über die Elektroden 11.1, 11.2 sowie ein Tastmodul 13.2 zum Abtasten einer Spannung über die Elektroden 11.1, 11.2. Die Impedanzmessung für das zweite Messsignal S2 wird somit über den Frequenzmodulator 13 der Impedanzmesseinheit 12 mittels einer Zweipolmessung umgesetzt, bei welcher die Spannungsabtastung über die gleichen Elektroden 11.1, 11.2 erfolgt wie die Aufprägung eines Stroms in das umliegende Gewebe. Dazu sind die Elektroden 11.1 und 11.2, nämlich in Form einer des Gehäusebehälters 52 und einer Stummelelektrode nahe zum Implantat 100 angeordnet. Das zweite Messsignal S2 wird in Form eines zweiten Impedanzsignals auf Basis einer zweiten Erregerfrequenz gebildet, die vergleichsweise niedrig - vorliegend bei 2 kHz - einen zeitlichen Impedanzverlauf wiedergibt, welcher die Durchblutung des das Implantat umgebenden Gewebes erfasst. Das Tastmodul 13.2 weist einen entsprechenden Tiefpass-Frequenzfilter auf, der in der Lage ist, das Impedanzsignal der niedrigeren Frequenz aus dem von den Elektroden 11.1, 11.2 erfassten Signal herauszufiltern. Außerdem ist der Frequenzmodulator 13 zur Ermittlung eines Hintergrundsignals ausgebildet, das in Form eines hochfrequenten Hintergrund-Impedanzsignals ermittelt wird. Dazu wird zusätzlich zum zweiten Impedanzsignal bei 2 kHz ein besonders hochfrequentes Hintergrund-Impedanzsignal bei vorliegend 1 MHz bestimmt. Die Variation der Frequenz der Impedanzmessung führt zu einem veränderten Kontrast zwischen Blut und Gewebe. Blut hat eine höhere Leitfähigkeit als das Gewebe. Dieser Abstand nimmt aber mit zunehmender Frequenz ab, so dass bei niedrigeren Frequenzen - vorliegend 2 kHz - ein höherer Kontrast zwischen Blut und Gewebe besteht als bei hohen Frequenzen. Vorliegend wird dieser Effekt genutzt, um rein geometrische Änderungen - wie etwaige Positionsverschiebungen der Elektroden 11.1, 11.2, Armbewegungen, Erschütterungen durch Gehen, Brustkorbbewegungen oder durch Atmung oder dergleichen - besser vom Volumenpuls der umgebenden Blutgefäße zu separieren. Die zwei Impedanzsignale bei 2 kHz und das hochfrequente Hintergrund-Impedanzsignal werden über den Frequenzmodulator 13 und die Elektroden 11.1, 11.2 gemessen und deren Differenz wird ausgewertet. Da geometrische Änderungen in das 2 kHz-Impedanzmesssignal und das 1 MHz-Hintergrund-Impedanzsignal gleich stark eingehen, während ein Blutpuls in dem thorakalen Gewebe in das 2 kHz-Impedanzsignal, also in die Messung bei niedriger Frequenz stärker eingeht, wird der Störeinfluss geometrischer Änderungen in der Differenz unterdrückt.

Ganz ähnlich - jedoch bei einer höheren Erregerfrequenz von vorliegend 20 kHz - funktioniert der Frequenzmodulator 14, welcher mit den Elektroden 11.3, 11.4 kontaktiert ist. Der Frequenzmodulator 14 weist ein Erregermodul 14.1 und ein Tastmodul 14.2 zum Auf prägen eines Stroms und Abtasten einer Spannung über die Elektroden 11.3, 11.4 auf. Die Impedanzmessung erfolgt mittels des Frequenzmodulators 14 und den entfernt zum Implantat 100 angeordneten Elektroden 11.3, 11.4 bei der höheren Erregerfrequenz von vorliegend 20 kHz. Damit wird das erste Messsignal S1 als ein zeitlicher Impedanzverlauf detektiert, welcher indikativ für einen Zeitpunkt eines Blutauswurfs aus dem Herzen ist, also dem kardialen Blutstrom B 1 zuzuordnen ist. Über die höhere Frequenz von 20 kHz im Vergleich zu der vorerwähnten niedrigeren Frequenz von 2 kHz ist sichergestellt, dass das erste Messsignal S 1 vorliegend Volumenänderungen des Herzens erfasst. Wiederum wird über den Frequenzmodulator 14 ein hochfrequentes Hintergrund-Impedanzsignal bei 1 MHz ermittelt. Die Differenz des 20 kHz-Impedanzsignals und des hochfrequenten Hintergrund-Impedanzmesssignals bei vorliegend 1 MHz separiert geometrische Änderungen der Elektrodenposition von Volumenänderungen des Herzens, da geometrische Änderungen in beide Messungen gleichstark eingehen, jedoch die Volumenänderung des Herzens stärker in die Messung bei niedrigerer Frequenz von 20 kHz.

Die Impedanzmesseinheit 12 enthält des Weiteren ein Zeittaktmodul 15, das ausgebildet ist, das erste Messsignal S 1 und das zweite Messsignal S2 zusammen mit den zugeordneten hochfrequenten Hintergrund-Impedanzmesssignalen zu alternieren. Mit anderen Worten erfolgt eine abwechselnde Messung des ersten Messsignals S 1 bei 20 kHz in Differenz zum Hintergrund-Impedanzmesssignal und des zweiten Messsignals S2 in Differenz zum weiteren Hintergrund-Impedanzmesssignal. Im Ergebnis entstehen erste und zweite Messsignale S1, S2, aus denen die an die Detektoranordnung 10 angeschlossene Überwachungsanordnung 20 in der Lage ist, einen ersten Parameter P1 - indikativ für einen Zeitpunkt eines Blutauswurfs aus dem Herzen - und einen zweiten Parameter - indikativ für einen Zeitpunkt eines Blutpulses in dem thorakalen Gewebe - zu erzeugen.

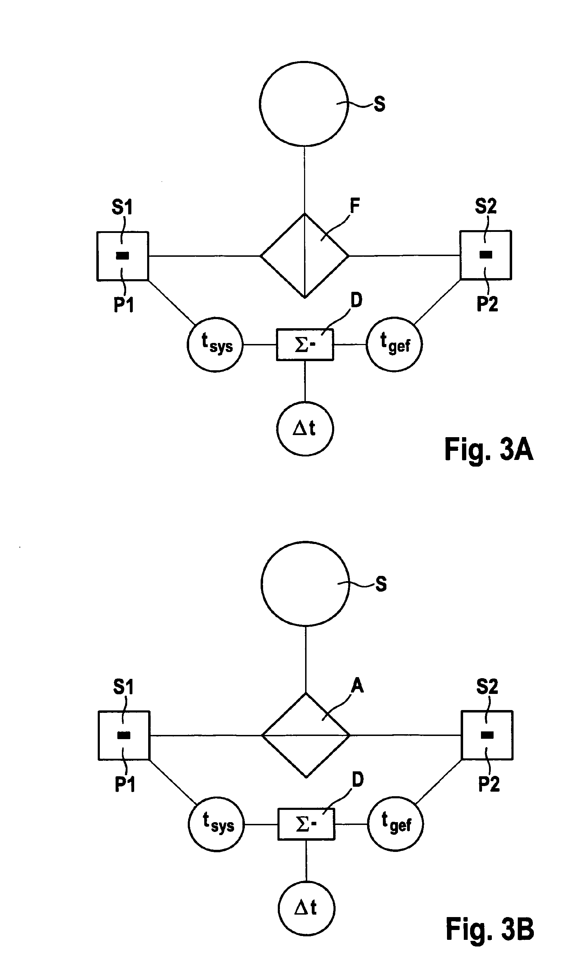

Dieses frequenzbasierte Impedanzmessverfahren zur Ermittlung des ersten und zweiten Messsignals S1, S2 in Form eines Impedanzsignals bei unterschiedlichen Frequenzen von vorliegend 20 kHz bzw. 2 kHz ist in Fig. 3A symbolisch dargestellt. Praktisch wird ein Impedanzmesssignal S durch einen in der Impedanzmesseinheit 12 enthaltenen Frequenzfilter F - als solcher in den Frequenzmodulatoren 13, 14 realisiert - in zwei Signalanteile S1, S2 aufgeteilt. Davon ist das erste Messsignal S1 bei vergleichsweise höherer Frequenz von vorliegend 20 kHz einem kardialen Blutstrom zugeordnet und zur Erzeugung eines für einen Zeitpunkt t_{sys} indikativen ersten Parameters P1 ausreichend. Das bei vergleichsweise niedriger Frequenz von vorliegend 2 kHz gebildete zweite Messsignal S2 ist zur Erzeugung eines für den Zeitpunkt eines Blutpulses im thorakalen peripheren Gewebe indikativen zweiten Parameters P2 ausreichend. Die aus den Parametern P1, P2 jeweils gebildeten Zeitpunkte t_{sys}, t_{gef} werden in einem Differenzbildner D voneinander subtrahiert und so die Pulswellenlaufzeit Δt gebildet.

In einer zur Ausführungsform der Fig. 2 und Fig. 3A variierten Ausführungsform kann, wie im Messverfahren der Fig. 3B gezeigt, der erste und zweite Parameter P1, P2 aus dem Zeitverlauf eines zeitlich transienten Impedanzsignals S gewonnen werden, indem anhand einer in einem Amplitudenfilter A gesetzten Amplitudenschwelle das transiente Impedanzsignal S in ein erstes Messsignal S 1 und ein zweites Messsignal S2 segmentiert wird. Dabei ist das erste Messsignal S 1 dem kardialen Blutstrom B 1 und das zweite Messsignal S2 dem epithorakalen peripheren Blutstrom B2 zugeordnet. Wiederum kann aus dem ersten Messsignal S1 der erste Parameter P1 und aus dem zweiten Messsignal S2 der zweite Parameter P2 gewonnen werden. Der Zeitpunkt t_{sys} eines Blutauswurfs aus dem Herzen und der Zeitpunkt t_{gef} eines Blutpulses in dem thorakalen Gewebe kann aus dem ersten bzw. zweiten Parameter P1, P2 mit einem geeigneten an sich bekannten Signalauswerte-Algorithmus ermittelt werden, so dass wiederum über einen Differenzbildner D die Differenz zwischen den Zeitpunkten t_{sys} und t_{gef} als Pulswellenlaufzeit Δt bestimmt werden kann.

Es ist zu verstehen, dass die vorgenannten Messsignale S1, S2 jeweils über das oben erläuterte hochfrequente Hintergrund-Impedanzsignal korrigierte Impedanzmesssignale darstellen.

Eine weitere Beseitigung von Hintergrundsignalen für das erste und zweite Messsignal kann dadurch erreicht werden, dass auf weiteren anderen Effekten beruhende Signalanteile weiteren unterschiedlichen Frequenzbereichen zugeordnet werden, die fern der eigentlichen Messfrequenzbereichen von vorliegend 2 kHz für das zweite Signal und 20 kHz für das erste Messsignal liegen. Dieses kann z.B. durch eine geeignete Signalmittelung und/oder durch eine bewegungssynchrone Signalverarbeitung erfolgen. So können beispielsweise gegenüber den Messfrequenzen von 2 kHz und 20 kHz vergleichsweise hochfrequente Signalanteile vorteilhaft ausgemittelt werden. Atemsynchrone Signalanteile oder andere vergleichsweise niederfrequente Signalanteile können beispielsweise durch eine synchrone Signalverarbeitung eliminiert werden.

Diese und andere Maßnahmen gelten auch für die Ermittlung nur des zweiten Messsignals S2 im Rahmen der anhand von Fig. 4 bis Fig. 6 erläuterten Ausführungsformen.

Fig. 4 zeigt eine zweite bevorzugte Ausfiihrungsform eines elektromedizinischen Implantats 101, bei welcher das zweite Messsignal S2, in analoger Weise zu Fig. 2 und Fig. 3A - alternativ Fig. 3B - ermittelt wird. Insofern ist folgend auf die Beschreibung zur Bildung des zweiten Messsignals S2 bei Fig. 2 bis Fig. 3B Bezug genommen. Im Unterschied zur dort beschriebenen ersten bevorzugten Ausführungsform wird das erste Messsignal S 1 bei der in Fig. 4 gezeigten zweiten Ausführungsform als EKG-Signal (Elektrokardiogramm-Signal) ermittelt. Dazu weist die Detektoranordnung 10` zusätzlich zu den Impedanzmesselektroden 11.1, 11.2 der Elektrodenanordnung 11 eine weitere Elektrodenanordnung 16 mit Elektroden 16.1, 16.2 auf und eine EKG-Messeinheit 17, die mit den EKG-Elektroden 16.1, 16.2 verbunden ist. Die R-Zacke des QRS-Komplexes im EKG-Signal wird vorliegend als erster Parameter P1 genutzt, welcher indikativ für einen Zeitpunkt eines Blutauswurfs aus dem Herzen ist, nämlich t_{sys}. Durch Differenzbildung von t_{sys} und dem aus dem zweiten Parameter P2 des zweiten Messsignals S2 in Form des Impedanzsignals ermittelten Zeitpunkt t_{gef} eines Blutpulses in dem thorakalen Gewebe wird wiederum die Überwachungsanordnung 20 und die Auswerteeinheit 30 genutzt, um die Pulswellenlaufzeit Δt zu ermitteln. Die Pulswellenlaufzeit Δt wird an das Kommunikationsmodul 40 übermittelt, welches die Pulswellenlaufzeit Δt an eine Empfangseinheit 200 des Überwachungssystems 1000 der Fig. 1 übermitteln kann.

Es ist zu verstehen, dass die Prinzipien der in Fig. 2 und Fig. 4 sowie Fig. 5 und Fig. 6 dargestellten Ausführungsformen nicht notwendigerweise alternativ realisiert werden müssen, sondern vielmehr ebenso miteinander kombinierbar sind. Mit anderen Worten kann vorliegend der erste Parameter P1 bzw. der Zeitpunkt t_{sys} des Blutauswurfs aus dem Herzen auf grund eines EKG-Signals gemäß Fig. 4 als auch eines kardialen Impedanzsignals gemäß Fig. 2 ermittelt werden. Beispielsweise könnte in einer geeigneten Kombination ein elektromedizinisches Implantat neben der Impedanzmesseinheit 12 des Implantats 100 auch eine EKG-Messeinheit 17 des Implantats 101 aufweisen.

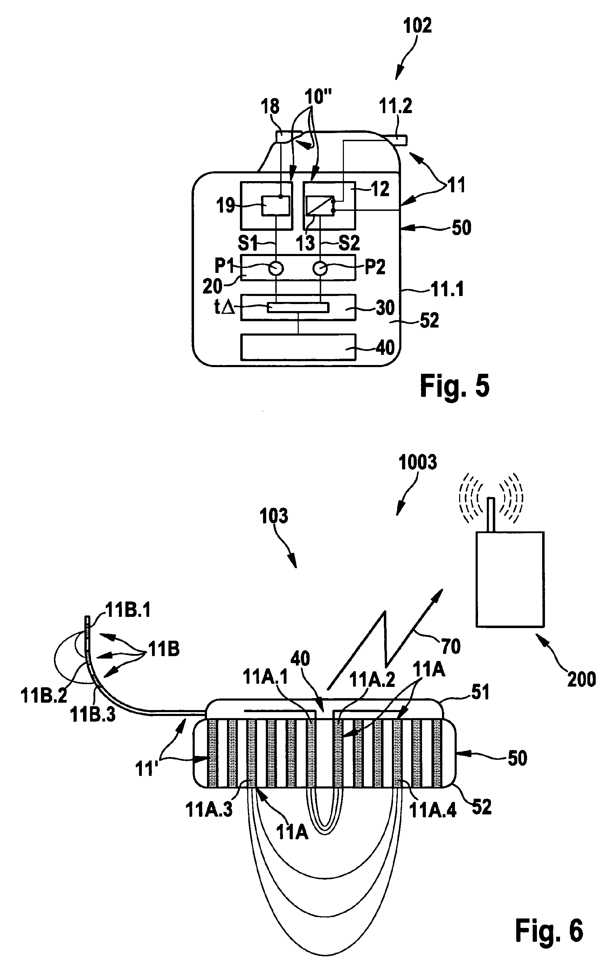

Fig. 5 zeigt eine weitere, dritte Ausfiihrungsform eines elektromedizinischen Implantats 102, bei dem die Detektoranordnung 10" eine Impedanzmesseinheit 12 mit einer Elektrodenanordnung 11 umfassend die Elektroden 11.1 und 11.2, aufweist. Die Detektoranordnung 10" ist eingerichtet, das zweite Messsignal S2 in Form eines Impedanzsignals zu detektieren. Insofern wird bezüglich der Ermittlung des zweiten Parameters P2 bzw. des zweiten Messsignals S2 und des Zeitpunkts t_{gef} auf die Beschreibung betreffend die Impedanzmesseinheit und den Frequenzmodulator 13 der Fig. 2 verwiesen. Vorliegend wird der Zeitpunkt t_{sys} eines Blutauswurfs aus dem Herzen aus einem ersten Parameter P des ersten Messsignals S 1 abgeleitet, wobei das erste Messsignal S 1 von einem akustischen Sensor 18 an eine Akustikmesseinheit 19 übermittelt wird, die zusammen in der Lage sind, Herztöne zu detektieren und daraus Klappenverschlussseiten des Herzens 1 zu bestimmen. In einer hier nicht gezeigten alternativen Ausführungsform könnte der Sensor 18 auch als ein Beschleunigungssensor ausgebildet sein, welcher den ventrikulären Blutauswurf direkt zu detektieren in der Lage ist. Das weitere Verfahren zur Bestimmung der Pulswellenlaufzeit Δt aus t_{sys} - abgeleitet aus dem ersten Parameter P1 - und t_{gef}- abgeleitet aus dem zweiten Parameter P2 - ergibt sich, wie bereits anhand von Fig. 1 beschrieben, nämlich in der dort erläuterten Weise aus dem Zusammenwirken der Überwachungsanordnung 20 und der Auswerteeinheit 30, welche die Pulswellenlaufzeit Δt schließlich an das Kommunikationsmodul 40 übermitteln.

Bei allen vorgenannten Ausführungsformen können zusätzliche Komponenten der Signalverarbeitung vorgesehen sein, um einen möglichst ungestörten auf den kardialen Blutstrom bzw. epithorakalen peripheren Blutstrom beschränktes erstes Messsignal S 1 und zweites Messsignal S2 zu generieren. So können zusätzliche Störungen oder Zeitintervalle mit unzuverlässigen Daten entweder unter Zuhilfenahme zusätzlicher Detektoren oder an sich bekannter Fehleralgorithmen identifiziert und bei der Bildung und/oder Auswertung des ersten und zweiten Messsignals S1, S2 berücksichtigt werden. Dabei hat sich insbesondere ein Beschleunigungssensor als besonders vorteilhaft erwiesen, um Messsignale nur während einer Ruheperiode des Patienten zu erfassen. Solche Messsignale haben sich als besonders störungsfrei erwiesen, da Atembewegungen oder sonstige Thoraxbewegungen des Patienten vergleichsweise amplitudengering ausfallen. In einer Weiterbildung dieses Ansatzes haben sich auch Maßnahmen als vorteilhaft erwiesen, bei denen Signalintervalle, die durch Störungen unbrauchbar sind, vollständig verworfen werden.

Zum Unterdrücken von Rauschen und geringen Störungen auf allen Messsignalanteilen können geeignete Filter- und Mittelungsmaßnahmen vorgesehen sein. Eine Mittelung von Zeitwerten, insbesondere von Zeitdifferenzen - vorteilhaft der Pulswellenlaufzeit selbst - über ausreichend viele Herzzyklen hat sich als grundsätzlich vorteilhaft erwiesen, um einen transienten Verlauf der Pulswellenlaufzeit und/oder eines Auswerteergebnisses zu glätten.

Darüber hinaus können Ableitungen erster oder höherer Ordnung sowie andere Parameterberechnungen nützlich sein, um Trends und/oder Trendparameter zu ermitteln oder als Signalanteil genauer erfassen zu können.

Physiologische Störungen unterliegen meistens einem bestimmten Rhythmus, wie z.B. die Atmung. Durch geeignete Mittelung oder Filterung, wie oben beschrieben, oder auch durch Triggerung auf bestimmte Atemphasen lassen sich Atembewegungen oder sonstige Bewegungen, insbesondere unter Nutzung eines Beschleunigungssensors, unterdrücken.

Besondere Umstände, wie Arrhythmien, hohe Herzfrequenzen oder gegebenenfalls Extrasystolen können außerdem durch Spezialsignalerkennungsmaßnahmen aus einem Messsignal extrahiert und entfernt werden.

Fig. 6 zeigt eine vierte bevorzugte Ausführungsform eines elektromedizinischen Implantats, das - grundsätzlich aufgebaut gemäß der ersten bevorzugten Ausführungsform, wie sie in Fig. 2 beschrieben ist - eine besonders bevorzugte alternative Elektrodenanordnung 11 aufweist. Fig. 6 zeigt dazu ein im Vergleich zu Fig. 1 variiertes Überwachungssystem 1003 mit einem variierten elektromedizinischen Implantat 103. Das elektromedizinische Implantat 103 weist einen inneren Aufbau ähnlich dem elektromedizinischen Implantat 100 auf. Wiederum ist ein Gehäuse 50 mit einem Gehäusebehälter 52, einem Header 51 sowie einer Elektrodenanordnung 11 vorgesehen. Das elektromedizinische Implantat 103 kann über ein in diesem Ausführungsbeispiel im Header 51 angeordnetes Kommunikationsmodul 40 eine drahtlose Telemetrieverbindung 70 zu einer Empfangseinheit 200 des Überwachungssystems 1003 aufbauen. Als Telemetrieeinheit im Kommunikationsmodul dient vorliegend eine Antenne, beispielsweise eine RF-Antenne oder eine sonstige den Patienten nicht beeinflussende Telemetrieverbindung. Ansonsten gelten hinsichtlich des Überwachungssystems 1003 die gleichen Anmerkungen wie in Bezug auf Fig. 1.

Die Elektrodenanordnung 11 weist vorliegend nahe zum Gehäuse 50 angeordnete Elektroden 11 A und entfernt an einem Kabel angeordnete Elektroden 11B auf. Die Elektroden 11A sind vorliegend als Streifenelektroden isoliert zum Gehäusebehälter 52 angebracht, wobei der Gehäusebehälter 52 vorliegend nicht als Elektrode dient. Die implantnahen Elektroden 11A dienen zusammen mit der Impedanzmesseinheit 12 zum Ermitteln des zweiten Messsignals S2, das einem epithorakalen peripheren Blutstrom B2 zugeordnet ist. Die implantatfernen Elektroden 11B dienen zusammen mit der Impedanzmesseinheit 12 zum Ermitteln eines ersten Messsignals S1, das einem kardialen Blutstrom B1 zugeordnet ist. Darüber hinaus gelten für die Elektroden 11A die Ausfiihrungen, wie sie bezüglich der Elektroden 11.1 und 11.2 in Fig. 2 gemacht wurden. Bezüglich der Elektroden 11B gelten die Ausführungen, wie sie im Bezug auf die Elektroden 11.3 und 11.4 der Fig. 2 gemacht wurden. Ein Impedanzmesssignal S, nämlich ein erstes Messsignal S1 und ein zweites Messsignal S2, lässt sich wiederum als Impedanzsignal S mit unterschiedlichen Frequenzanteilen mit den Frequenzmodulatoren 13, 14 in einer in Fig. 6 nicht näher gezeigten Impedanzmesseinheit 12 ermitteln.

Vorliegend sind die Elektroden 11 A als Streifenelektroden ausgeführt. In einer hier nicht gezeigten variierten Ausbildungsform können die Elektroden 11 A auch als Ringelektroden ausgeführt sein. Ringelektroden sind streifenartige Elektroden, die um den gesamten Gehäusebehälter 52 herumgeführt sind, sie diesen also vollständig umgeben. Streifenelektroden sind vorliegend lediglich auf einer Seite oder auf zwei gegenüberliegenden Seiten des Gehäusebehälters 52 angeordnet, ohne dass sich gegenüberliegende bzw. nebeneinander liegende Streifenelektroden kontaktieren. Durch geeignete Verbindung bzw. Schaltung der Streifenelektroden 11A untereinander können jedoch Paare von Streifenelektroden - z.B. ein Paar von Streifenelektroden 11A.1 und 11A.2 - zum Zwecke einer Zweipolmessung gekoppelt werden, um einen Strom zur Messung einer thorakalen Leitfähigkeit bei vergleichsweise niedriger Frequenz - vorliegend wiederum bei 2 kHz - genutzt werden. Da das Paar von Elektroden 11A.1 und 11A.2 vergleichsweise nahe beieinander liegt, hat das Einflussgebiet des aufgeprägten Stroms bzw. der detektierten Spannung vergleichsweise geringe Abmessungen im umliegenden Gewebe des Implantats 103. Dagegen kann eine ähnliche paarweise Schaltung von Elektroden 11A.3 und 11A.4 ein Einflussgebiet des umliegenden Gewebes mit vergleichsweise größeren Abmessungen erfassen. Weitere Elektroden - außer den Elektroden 11A - können mit unterschiedlich variierten Abständen paarweise, zusammengefasst werden. So können verschiedene Messkonfigurationen allein oder in beliebiger Kombination gleichzeitig sequentiell oder alternierend über die Impedanzmesseinheit 12 eingestellt werden und Impedanzsignale mit unterschiedlichem Informationsgehalt bzw. mit unterschiedlichem Einflussgebiet des Gewebes, gemessen werden. Dies kann sich als sinnvoll erweisen, wenn z.B. eine Redundanz für einen zweiten Parameter P2 aus dem zweiten Messsignal S2 aus einer Anzahl von zweiten Messsignalen erzeugt werden soll, wobei das zweite Messsignal S2 oder die Anzahl von zweiten Messsignalen indikativ für einen Zeitpunkt eines Blutpulses in dem thorakalen Gewebe ist. Analog können von den Elektroden 11B unterschiedliche Elektroden, z.B. die Elektroden 11B.1 und die Elektroden 11B.2 paarweise zusammengeschaltet werden, um einen engen distalen kardialen Impedanzverlauf als erstes Messsignal S zu erfassen. Alternativ können die drei distalen Elektroden 11B.1, 11B.2, 11B.3 auch zum Erfassen eines EKG-Signals und dessen Ableitungen genutzt werden.

Insgesamt bietet die Auslegung der Elektrodenanordnung 11 bei dem Implantat 103 der Fig. 6 die Möglichkeit, mittels Kopplung von Elektroden unterschiedlicher Distanz zu Polpaaren, ein Impedanzsignal aus unterschiedlichen Einflussgebieten - nämlich mit geringen oder weiterreichenden Abmessungen im Gewebe - zu erhalten.

Das vorliegende Beispiel wurde anhand einer Zweipolmessung erläutert, bei welcher eine Spannungsmessung und eine Stromaufprägung mit gleichen Elektroden erfolgt. Grundsätzlich können Messungen auch so ausgeführt werden, dass unterschiedliche Elektroden zur Spannungsabtastung und Stromaufprägung genutzt werden oder teilweise gemeinsam genutzt werden.

Zusammenfassend betrifft die Erfindung ein elektromedizinisches Implantat 100, 101, 102, 103 zum Überwachen eines kardialen Blutstroms B1 und eines epithorakalen, peripheren Blutstroms B2 eines Lebewesens, aufweisend:
- eine Detektoranordnung 10, 10', 10", die eingerichtet ist zum Erfassen eines dem kardialen Blutstrom B zugeordneten ersten Messsignals S 1 und eines dem epithorakalen, peripheren Blutstroms B2 zugeordneten zweiten Messsignals S2;
- eine an der Detektoranordnung 10, 10', 10" angeschlossene Überwachungsanordnung 20, die
   eingerichtet ist, einen ersten Parameter P1 aus dem ersten Messsignal S1 zu erzeugen, der indikativ für einen Zeitpunkt t_{sys} eines Blutauswurfs aus dem Herzen ist, und eingerichtet ist, einen zweiten Parameter P2 aus dem zweiten Messsignal S2 zu erzeugen, der indikativ für einen Zeitpunkt t_{gef} eines Blutpulses in dem thorakalen Gewebe ist, der dem Blutauswurf zugeordnet ist;
- eine an der Überwachungsanordnung 20 angeschlossene Auswerteeinheit 30, die eingerichtet ist, eine Pulswellenlaufzeit Δt=t_{gef}-t_{sys} aus dem ersten Parameter P1 und dem zweiten Parameter P2 zu erzeugen. Dabei ist vorgesehen, dass die Detektoranordnung 10, 10', 10" eine Impedanzmesseinheit 12 mit einer Elektrodenanordnung 11, 11' umfasst, die eingerichtet sind, wenigstens das zweite Messsignal S2 in Form eines Impedanzsignals S zu detektieren.

### Bezugszeichenliste

- 1: Herz
- 2: Thorax
- 10, 10', 10": Detektoranordnung
- 11, 11 `: Elektrodenanordnung
- 11.1, 11A.1, 11B.1: Elektrode
- 11.2, 11A.2, 11B.2: Elektrode
- 11.3, 11A.3, 11B.3: Elektrode
- 11.4, 11A.4: Elektrode
- 11 A: Elektrode
- 11 B: Elektrode
- 12: Impedanzmesseinheit
- 13, 14: Frequenzmodulator
- 13.1, 14.1: Erregermodul
- 13.2, 14.2: Tastmodul
- 15: Zeittaktmodul
- 16: Elektrodenanordnung
- 16.1, 16.2: Elektrode
- 17: EKG-Messeinheit
- 18: akustischer Detektor
- 19: Akustikmesseinheit
- 20: Überwachungsanordnung
- 30: Auswerteeinheit
- 40: Kommunikationsmodul
- 50: Gehäuse
- 51: Header
- 52: Gehäusebehälter
- 53: Verankerungsmittel
- 60: Elektronik
- 70: Telemetrieverbindung
- 100, 101, 102, 103: elektromedizinisches Implantat
- 200: Empfangseinheit
- 300: Speichereinheit
- 1000: Überwachungssystem
- 1003: Überwachungssystem

- A: Amplitudenfilter
- B1: kardialer Blutstrom
- B2: epithorakaler peripherer Blutstrom
- D: Differenzbildner
- F: Frequenzfilter
- P1: erster Parameter
- P2: zweiter Parameter
- S: Impedanzmesssignal
- S1: erstes Messsignal
- S2: zweites Messsignal
- Δt: Pulswellenlaufzeit
- t_{gef}: Zeitpunkt eines Blutpulses im thorakalen Gewebe
- t_{sys}: Zeitpunkt eines Blutauswurfes aus dem Herzen

## Patentansprüche

1. Elektromedizinisches Implantat (100, 101, 102, 103) zum Überwachen eines kardialen Blutstroms (B1) und eines epithorakalen, peripheren Blutstroms (B2) eines Lebewesens, aufweisend:
- eine Detektoranordnung (10, 10', 10"), die eingerichtet ist zum Erfassen eines dem kardialen Blutstrom (B1) zugeordneten ersten Messsignals (S1) und eines dem epithorakalen, peripheren Blutstrom (B2) zugeordneten zweiten Messsignals (S2);
- eine an der Detektoranordnung (10, 10', 10") angeschlossene Überwachungsanordnung (20), die
eingerichtet ist, einen ersten Parameter (P1) aus dem ersten Messsignal (S1) zu erzeugen, der indikativ für einen Zeitpunkt (t_{sys}) eines Blutauswurfs aus dem Herzen ist, und
eingerichtet ist, einen zweiten Parameter (2) aus dem zweiten Messsignal (S2) zu erzeugen, der indikativ für einen Zeitpunkt (t_{gef}) eines Blutpulses in dem thorakalen Gewebe ist, der dem Blutauswurf zugeordnet ist;
- eine an der Überwachungsanordnung (20) angeschlossene Auswerteeinheit (30), die eingerichtet ist, eine Pulswellenlaufzeit (Δt) aus dem ersten Parameter (P1) und dem zweiten Parameter (P2) zu erzeugen;
wobei
die Detektoranordnung (10, 10', 10") eine Impedanzmesseinheit (12) mit einer Elektrodenanordnung (11, 11') umfasst, die eingerichtet sind, wenigstens das zweite Messsignal (S2) in Form eines Impedanzsignals (S) zu detektieren.

2. Implantat nach Anspruch 1, weiter aufweisend ein Kommunikationsmodul (40) zur drahtlosen Übermittlung der Pulswellenlaufzeit (Δt) und/oder eines von der Auswerteeinheit (30) auf Grundlage der Pulswellenlaufzeit (Δt) gebildeten Auswerteergebnisses.

3. Implantat nach Anspruch 1 oder 2 **dadurch gekennzeichnet, dass** die Elektrodenanordnung (11, 11') eine Anzahl von Polen ausbildet über die mit der Impedanzmesseinheit (12) eine Anzahl von Impedanzsignalen (S) detektierbar sind.

4. Implantat nach einem der Ansprüche 1 bis 3 **dadurch gekennzeichnet, dass** die Impedanzmesseinheit (12) und die Elektrodenanordnung (11, 11') eingerichtet sind, das erste Messsignal (S1) in Form eines Impedanzsignals (S) zu detektieren.

5. Implantat nach einem der Ansprüche 1 bis 4 **dadurch gekennzeichnet, dass** die Elektrodenanordnung (11, 11') wenigstens eine nahe zum des Implantat (100, 101, 102, 103) angeordnete Elektrode (11.1, 11.2, 11A.1, 11A.2, 11A.3, 11A.4) umfasst und/oder die Elektrodenanordnung (11, 11') wenigstens eine entfernt zum Implantat (100, 101, 102, 103) angeordnete weitere Elektrode (11.3, 11.4, 11B.1, 11B.2, 11B.3) umfasst.

6. Implantat nach Anspruch 5 **dadurch gekennzeichnet dass** die nahe zum Implantat (100, 101, 102, 103) angeordnete Elektrode in Form eines Gehäusebehälters (r2) des Implantats gebildet ist.

7. Implantat nach einem der Ansprüche 1 bis 6 **dadurch gekennzeichnet, dass** die Impedanzmesseinheit (12) ein Erregermodul (13.1, 14.1) zum Abgeben eines elektrischen Stroms über die Elektrodenanordnung (11, 11') und ein Tastmodul (13.2, 14.2) zum Annehmen einer Spannung über die Elektrodenanordnung (11, 11') aufweist.

8. Implantat nach einem der Ansprüche 1 bis 7 **dadurch gekennzeichnet, dass** das zweite Messsignal (S2) in Form eines zweiten Impedanzsignals auf Basis einer zweiten Erregerfrequenz und/oder das erste Messsignal (S1) in Form eines ersten Impedanzsignals auf Basis einer ersten Erregerfrequenz gebildet ist.

9. Implantat nach Anspruch 8 **dadurch gekennzeichnet, dass** die zweite Erregerfrequenz niedriger als die erste Erregerfrequenz ist.

10. Implantat nach einem der Ansprüche 1 bis 9 **dadurch gekennzeichnet, dass** die Detektoreinheit (10, 10', 10") ein Zeittaktmodul (15) umfasst, das ausgebildet ist, das zweite Messsignal (S2) und das erste Messsignal (S1) zu sequenzieren und/oder zu alternieren.

11. Implantat nach einem der Ansprüche 1 bis 10 **dadurch gekennzeichnet, dass** die Detektoreinheit (10, 10', 10") einen Frequenzmodulator (13, 14) aufweist, der ausgebildet ist, ein Hintergrundsignal in Form eines hochfrequenten Hintergrund-Impedanzmesssignals zu ermitteln, wobei die Erregerfrequenz des hochfrequenten Hintergrund-Impedanzmesssignals über einer ersten und/oder zweiten Erregerfrequenz liegt.

12. Implantat nach einem der Ansprüche 1 bis 11 **dadurch gekennzeichnet, dass** das zweite Messsignal (S2) ein über eine Amplitudenschwelle separierbares Teilsegment eines transienten Impedanzmesssignals (S) ist, und/oder das erste Messsignal (S1) ein über eine Amplitudenschwelle separierbares Teilsegment eines transienten Impedanzmesssignals (S) ist.

13. Implantat nach einem der Ansprüche 1 bis 12 **dadurch gekennzeichnet, dass** die Elektrodenanordnung (11, 11') symmetrisch zu einem Gehäusebehälter (52) des Implantats (100, 101, 102, 103) und dabei mehrseitig oder allseitig oder ringförmig um einen Gehäusebehälter (52) des Implantats angeordnet sind.

14. Implantat nach einem der Ansprüche 1 bis 13 **dadurch gekennzeichnet, dass** die Detektoranordnung (10, 10', 10") zum Erfassen des ersten Messsignals (S1) umfasst: eine EKG-Messeinheit (17) mit einer Elektrodenanordnung (16), die eingerichtet sind das erste Messsignal (S1) in Form eines (Elektrokardiogramm-) EKG-Signals zu detektieren.

15. Implantat nach einem der Ansprüche 1 bis 14 die Detektoranordnung (10, 10', 10") eingerichtet ist zum Erfassen des zweiten Messsignals (S2) und/oder des ersten Messsignals (S1) und einen oder mehrere der Detektoren umfasst, die ausgewählt sind aus der Gruppe bestehend aus: akustischem Detektor (18), optischem Detektor, Druck- und/oder Dehnungs-Detektor, Beschleunigungsdetektor.

16. Überwachungssystem (1000, 1003) aufweisend das elektromedizinische Implantat (100, 101, 102, 103) nach einem der vorhergehenden Ansprüche, wobei das Implantat
- ein Kommunikationsmodul (40) zur drahtlosen Übermittlung der Pulswellenlaufzeit (Δt) und/oder eines von der Auswerteeinheit auf Grundlage der Pulswellenlaufzeit (Δt) gebildeten Auswerteergebnisses aufweist, weiter aufweisend:
- eine Empfangseinheit (200) zum Empfangen der Pulswellenlaufzeit (Δt) und/oder des Auswerteergebnisses;
- eine Speichereinheit (300) für die Pulswellenlaufzeit (Δt) und/oder das Auswerteergebnis.

## Claims

1. An electromedical implant (100, 101, 102, 103) for monitoring a cardiac blood flow (B1) and an epithoracic, peripheral blood flow (B2) of a living being, said implant having:
- a detector arrangement (10, 10', 10"), which is configured to capture a first measurement signal (S1) associated with the cardiac blood flow (B1) and a second measurement signal (S2) associated with the epithoracic, peripheral blood flow (B2);
- a monitoring arrangement (20), which is connected to the detector arrangement (10, 10', 10"), which
is configured to generate a first parameter (P1) from the first measurement signal (S1), said parameter being indicative of a time (t_{sys}) at which blood is ejected from the heart, and which
is configured to generate a second parameter (P2) from the second measurement signal (S2), said parameter being indicative of a time (t_{gef}) of a blood pulse in the thoracic tissue associated with the ejection of blood;
- an evaluation unit (30), which is connected to the monitoring arrangement (20) and which is configured to generate a pulse transit time (At) from the first parameter (P1) and the second parameter (P2);
wherein
the detector arrangement (10, 10', 10") comprises an impedance measurement unit (12) having an electrode arrangement (11, 11'), which are configured to detect at least the second measurement signal (S2) in the form of an impedance signal (S).

2. The implant according to Claim 1, further comprising a communication module (40) for wirelessly transmitting the pulse transit time (At) and/or an evaluation result formed by the evaluation unit (30) on the basis of the pulse transmit time (At).

3. The implant according to Claim 1 or 2, **characterised in that** the electrode arrangement (11, 11') forms a number of poles, via which a number of impedance signals (S) can be detected using the impedance measurement unit (12).

4. The implant according to one of Claims 1 to 3, **characterised in that** the impedance measurement unit (12) and the electrode arrangement (11, 11') are configured to detect the first measurement signal (S1) in the form of an impedance signal (S).

5. The implant according to one of Claims 1 to 4, **characterised in that** the electrode arrangement (11, 11') comprises at least one electrode (11.1, 11.2, 11A.1, 11A.2, 11A.3, 11A.4) arranged close to the implant (100, 101, 102, 103) and/or the electrode arrangement (11, 11') comprises at least one further electrode (11.3, 11.4, 11B.1, 11B.2, 11B.3) arranged at a distance from the implant (100, 101, 102, 103).

6. The implant according to Claim 5, **characterised in that** the electrode arranged close to the implant (100, 101, 102, 103) is formed as a housing container (r2) of the implant.

7. The implant according to one of Claims 1 to 6, **characterised in that** the impedance measurement unit (12) has an excitation module (13.1, 14.1) for delivering an electrical current via the electrode arrangement (11, 11'), and has a sensing module (13.2, 14.2) for receiving a voltage via the electrode arrangement (11, 11').

8. The implant according to one of Claims 1 to 7, **characterised in that** the second measurement signal (S2) is formed as a second impedance signal on the basis of a second excitation frequency and/or the first measurement signal (S1) is formed as a first impedance signal on the basis of a first excitation frequency.

9. The implant according to Claim 8, **characterised in that** the second excitation frequency is lower than the first excitation frequency.

10. The implant according to one of Claims 1 to 9, **characterised in that** the detector unit (10, 10', 10") comprises a timing module (15), which is configured to sequence and/or to alternate the second measurement signal (S2) and the first measurement signal (S1).

11. The implant according to one of Claims 1 to 10, **characterised in that** the detector unit (10, 10', 10") has a frequency modulator (13, 14), which is configured to determine a background signal in the form of a high-frequency background impedance measurement signal, wherein the excitation frequency of the high-frequency background impedance measurement signal lies above a first and/or second excitation frequency.

12. The implant according to one of Claims 1 to 11, **characterised in that** the second measurement signal (S2) is a sub-segment of a transient impedance measurement signal (S), which sub-signal can be separated via an amplitude threshold, and/or the first measurement signal (S1) is a sub-segment of a transient impedance measurement signal (S), which sub-signal can be separated via an amplitude threshold.

13. The implant according to one of Claims 1 to 12, **characterised in that** the electrode arrangement (11, 11') is arranged symmetrically with respect to a housing container (52) of the implant (100, 101, 102, 103), moreover on a number of sides or on all sides or annularly around a housing container (52) of the implant.

14. The implant according to one of Claims 1 to 13, **characterised in that** the detector arrangement (10, 10', 10") for capturing the first measurement signal (S1) comprises: an ECG measurement unit (17) having an electrode arrangement (16), which are configured to detect the first measurement signal (S1) in the form of an (electrocardiogram) ECG signal.

15. The implant according to one of Claims 1 to 14, the detector arrangement (10, 10', 10") being configured to capture the second measurement signal (S2) and/or the first measurement signal (S1) and comprising one or more detectors selected from the group consisting of: acoustic detector (18), optical detector, pressure and/or expansion detector, and acceleration detector.

16. A monitoring system (1000, 1003) comprising the electromedical implant (100, 101, 102, 103) according to one of the preceding claims, wherein the implant comprises
- a communication module (40) for wirelessly transmitting the pulse transit time (At) and/or an evaluation result formed by the evaluation unit on the basis of the pulse transit time (At), and further comprising:
- a receiving unit (200) for receiving the pulse transit time (At) and/or the evaluation result;
- a memory unit (300) for the pulse transit time (At) and/or the evaluation result.

## Revendications

1. Implant électromédical (100, 101, 102, 103) pour la surveillance d'un flux sanguin cardiaque (B1) et d'un flux sanguin périphérique de l'épithorax (B2) d'un être vivant, présentant :
- un agencement de détecteurs (10, 10', 10") qui est installé pour la saisie d'un premier signal de mesure (S1) associé à un flux sanguin cardiaque (B1) et d'un deuxième signal de mesure (S2) associé à un flux sanguin périphérique de l'épithorax (B2) ;
- un ensemble de surveillance (20) associé à l'agencement de détecteurs (10, 10', 10") qui
est installé pour générer un premier paramètre (P1) à partir du premier signal de mesure (S1) qui est indicateur pour un temps (t_{sys}) d'une expulsion de sang partant du coeur, et
est installé pour générer un deuxième paramètre (2) à partir du deuxième signal de mesure (S2) qui est indicateur pour un temps (t_{gef}) d'une impulsion de sang dans le tissu thoracique, qui est associée à l'expulsion de sang ;
- une unité d'évaluation (30) associée à l'ensemble de surveillance (20) qui est installée pour générer une période d'impulsion (Δt) à partir du premier paramètre (P1) et du deuxième paramètre (P2) ;
où
l'agencement de détecteurs (10, 10', 10") comprend une unité de mesure d'impédance (12) avec un agencement d'électrodes (11, 11'), qui est installée pour détecter au moins le deuxième signal de mesure (S2) sous la forme d'un signal d'impédance (S).

2. Implant selon la revendication 1, présentant en outre un module de communication (40) pour la transmission sans fil de la période d'impulsion (Δt) et/ou d'un résultat d'évaluation élaboré sur la base de la période d'impulsion (Δt) par l'unité d'évaluation (30).

3. Implant selon les revendications 1 ou 2, **caractérisé en ce que** l'agencement d'électrodes (11, 11') est formé par un nombre de pôles par lesquels un nombre de signaux d'impédance (S) peuvent être détectés avec l'unité de mesure d'impédance (12).

4. Implant selon l'une des revendications 1 à 3, **caractérisé en ce que** l'unité de mesure d'impédance (12) et l'agencement d'électrodes (11, 11') sont installés pour détecter le premier signal de mesure (S1) sous la forme d'un signal d'impédance (S).

5. Implant selon l'une des revendications 1 à 4, **caractérisé en ce que** l'agencement d'électrodes (11, 11') comprend au moins une électrode (11.1, 11.2, 11A.1, 11A.2, 11A.3, 11A.4) disposée près de l'implant (100, 101, 102,103) et/ou l'agencement d'électrodes (11, 11') comprend au moins une autre électrode (11.3, 11.4, 11B.1, 11B.2, 11B.3) éloignée par rapport à l'implant (100, 101, 102, 103).

6. Implant selon la revendication 5, **caractérisé en ce que** l'électrode disposée près de l'implant (100, 101, 102, 103) est conçue sous la forme d'un récipient de boîtier (r2) de l'implant.

7. Implant selon l'une des revendications 1 à 6, **caractérisé en ce que** l'unité de mesure d'impédance (12) présente un module d'excitation (13.1, 14.1) pour la délivrance d'un courant électrique par l'intermédiaire de l'agencement d'électrodes (11, 11') et un module interrupteur à clé (13.2, 14.2) pour l'admission d'une tension par l'intermédiaire de l'agencement d'électrodes (11, 11').

8. Implant selon l'une des revendications 1 à 7, **caractérisé en ce que** le deuxième signal de mesure (S2) est élaboré sous la forme d'un deuxième signal d'impédance sur la base de la deuxième fréquence d'excitation et/ou le premier signal de mesure (S1) est élaboré sous la forme d'un premier signal d'impédance sur la base d'une première fréquence d'excitation.

9. Implant selon la revendication 8, **caractérisé en ce que** la deuxième fréquence d'excitation est plus faible que la première fréquence d'excitation.

10. Implant selon l'une des revendications 1 à 9, **caractérisé en ce que** l'agencement de détecteurs (10, 10',10") comprend un module de minutage (15) qui est conçu pour séquencer et/ou alterner le deuxième signal de mesure (S2) et le premier signal de mesure (S1).

11. Implant selon l'une des revendications 1 à 10, **caractérisé en ce que** l'agencement de détecteurs (10, 10', 10") comprend un modulateur de fréquence (13, 14) qui est conçu pour déterminer un signal de ligne de base sous la forme d'un signal d'impédance de ligne de base à fréquence élevée, où la fréquence d'excitation du signal de mesure d'impédance de la ligne de base à fréquence élevée se situe au dessus d'une première, et/ou d'une deuxième fréquence d'excitation.

12. Implant selon l'une des revendications 1 à 11, **caractérisé en ce que** le deuxième signal de mesure (S2) est un segment partiel d'un signal de mesure d'impédance (S) transitoire pouvant être séparé par un seuil d'amplitude, et/ou le premier signal de mesure (S1) est un segment partiel d'un signal de mesure d'impédance (S) transitoire pouvant être séparé par un seuil d'amplitude.

13. Implant selon l'une des revendications 1 à 12, **caractérisé en ce que** l'agencement d'électrodes (11, 11') est disposé symétriquement par rapport à un récipient de boîtier (52) de l'implant (100, 101, 102, 103) et de ce fait est disposé sur plusieurs côtés ou sur tous les côtés, ou sous la forme d'un anneau entourant le récipient de boîtier (52) de l'implant.

14. Implant selon l'une des revendications 1 à 13, **caractérisé en ce que** l'agencement des détecteurs (10, 10', 10") comprend, pour la détection du premier signal de mesure (S1) : une unité de mesure d'ECG (17) avec un arrangement d'électrodes (16) qui est installé pour détecter le premier signal de mesure (S1) sous la forme d'un signal d'ECG (électrocardiogramme).

15. Implant selon l'une des revendications 1 à 14, l'agencement des détecteurs (10, 10', 10") étant installé pour la saisie du deuxième signal de mesure (S2) et/ou du premier signal de mesure (S1) et comprenant un ou plusieurs détecteurs, qui sont choisis dans le groupe constitué par : un détecteur acoustique (18), un détecteur optique, un détecteur sensible à la pression et/ou à la traction, un détecteur de vitesse.

16. Système de surveillance (1000, 1003) présentant l'implant électromédical (100, 101, 102, 103) selon l'une des revendications précédentes, dans lequel l'implant présente
- un module de communication (40) pour la transmission sans fil de la période d'impulsion (Δt) et/ou un résultat d'évaluation élaboré par l'unité d'évaluation sur la base de la période d'impulsion (Δt), comprenant en outre :
- une unité de réception (200) pour la réception de la période d'impulsion (Δt) et/ou du résultat de l'évaluation ;
- une unité de stockage (300) pour la période d'impulsion (Δt) et/ou du résultat de l'évaluation.
